(19) Europäisches Patentamt
European Patent Office
Office européen des brevets

(11) Veröffentlichungsnummer: **0 320 448 B1**

(12) **EUROPÄISCHE PATENTSCHRIFT**

(45) Veröffentlichungstag der Patentschrift: **28.12.94**

(51) Int. Cl.$^5$: **C07D 213/64**, C07D 213/74, C07D 213/70, A01N 47/34, //C07D213/61,C07C265/12, C07C233/10

(21) Anmeldenummer: **88810818.0**

(22) Anmeldetag: **29.11.88**

# (54) 3-Aminobenzoylphenylharnstoffe.

(30) Priorität: **07.12.87 CH 4756/87**

(43) Veröffentlichungstag der Anmeldung: **14.06.89 Patentblatt 89/24**

(45) Bekanntmachung des Hinweises auf die Patenterteilung: **28.12.94 Patentblatt 94/52**

(84) Benannte Vertragsstaaten: **AT BE CH DE FR GB IT LI LU NL SE**

(56) Entgegenhaltungen:
**EP-A- 0 079 311**
**EP-A- 0 185 621**
**DE-A- 3 241 138**
**US-A- 3 450 747**

**BULLETIN OF THE ACADEMY OF SCIENCES OF THE USSR, DIVISION OF CHEMICAL SCIENCE,Band 36, Nr. 4, Teil 2, 20. Oktober 1987, Seiten 761-765; I.A. RYBAKOVA et al.:"Photostimulated and dark reactions of aryl halides with 2- and 4-pyridine-thiolates"**

(73) Patentinhaber: **CIBA-GEIGY AG**
**Klybeckstrasse 141**
**CH-4002 Basel (CH)**

(72) Erfinder: **Maienfisch, Peter, Dr.**
**Traugott Meyer-Strasse 5**
**CH-4147 Aesch (CH)**
Erfinder: **Gehret, Jean-Claude, Dr.**
**Im Aeschfeld 12**
**CH-4147 Aesch (CH)**
Erfinder: **Frei, Bruno, Dr.**
**Lippestrasse 14**
**CH-4415 Lausen (CH)**

EP 0 320 448 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 93, 1980, Seite 586, Zusammenfassung Nr. 131594q,Columbus, Ohio, US; M.B. MOHAMED et al.: "Preparation of all the monochloro-alpha-carbolines and assignment of the carbon-13 NMR spectrum of alpha-carboline"**

**TETRAHEDRON, Band 27, Nr. 24, Dezember 1971, Seiten 6011-6021, Pergamon Press;T. MIZUNO et al.: "Studies on the conformational isomers of 2-anilinopyridinesand related compounds [arylaminoquinolines; IR; UV; Rotational isomerism;tautomerism]**

**CHEMICAL ABSTRACTS, Band 83, 1975, Seite 473, Zusammenfassung Nr. 27831x,Columbus, Ohio, US; J. ROMANOWSKI et al.: "Use of 2,6-dichlorobenzonitrile(dichlobenil) as a starting substance for synthesizing herbicides derived frombenzoic acid"**

## Beschreibung

Die vorliegende Erfindung betrifft neue Benzoylphenylharnstoffe, welche im Benzoylteil in 3-Stellung eine substituierte oder unsubstituierte Aminogruppe besitzen, Verfahren zu ihrer Herstellung und ihre Verwendung in der Schädlingsbekämpfung. Neue Ausgangsstoffe und deren Herstellung bilden ebenfalls einen Gegenstand der Erfindung.

Die erfindungsgemässen substituierten 3-Aminobenzoylphenylharnstoffe haben die Formel I

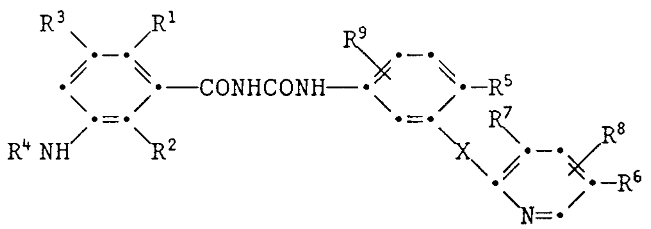

(I)

worin

| | |
|---|---|
| $R^1$, $R^2$, $R^3$, $R^7$, $R^8$ und $R^9$ | unabhängig voneinander H oder Halogen, |
| $R^4$ | H, $R^{10}$CO- oder $R^{11}$NHCO-, wobei $R^{10}$ für ein $C_1$-$C_4$-Alkyl steht, welches unsubstituiert oder mit einem bis drei gleichen oder verschiedenen Substituenten aus der Gruppe Halogen, $C_1$-$C_4$-Alkoxy, $C_1$-$C_4$-Acyloxy und -COOG substituiert ist, worin G H, ein Alkali-oder Erdalkalimetallkation bedeutet, und $R^{11}$ für eine unsubstituierte oder durch Halogen substituierte $C_1$-$C_4$-Alkyl- oder Phenylgruppe steht, |
| $R^5$ und $R^6$ | unabhängig voneinander H, Halogen, $C_1$-$C_6$-Alkyl oder $C_1$-$C_6$-Haloalkyl und |
| X | O, $S(O)_n$ oder NH, wobei n für 0, 1 oder 2 steht, bedeuten. |

Im Rahmen der vorliegenden Erfindung sind unter Halogen als Substituent oder Teil eines Substituenten Fluor, Chlor, Brom und Jod zu verstehen, vorzugsweise Fluor, Chlor und Brom und insbesondere Fluor und Chlor.

Alkylgruppen als Substituenten oder Teil eines Substituenten können geradkettig oder verzweigt sein. Sofern keine andere Definition gegeben ist, steht Alkyl vorzugsweise für $C_1$-$C_6$-Alkyl, beispielsweise Methyl, Ethyl, Propyl, Isopropyl, Butyl, Isobutyl, sek.-Butyl, tert.-Butyl, n-Pentyl und n-Hexyl. Bevorzugt werden Alkylgruppen mit 1 bis 4 Kohlenstoffatomen, insbesondere Methyl und Ethyl.

Als Haloalkylgruppen kommen halogenierte Alkylreste in Frage, worin der Alkylrest den vorstehend angegebenen Definitionen entspricht und teilweise oder vollständig halogeniert ist. Beispiele für solche Gruppen sind $CF_3$, $CCl_3$, $C_2H_4F$, $CF_2CCl_2F$, vorzugsweise $CF_3$ und $CF_2CCl_2F$, insbesondere $CF_3$.

Unter $C_1$-$C_4$-Acyloxy sind Reste aus der Gruppe $C_1$-$C_3$-Alkyl-COO-, $C_1$-$C_3$-Alkenyl-COO-, $C_1$-$C_3$-Alkinyl-COO- und Cyclopropyl-COO- zu verstehen. Alkyl kann Methyl, Ethyl oder Propyl, Alkenyl kann beispielsweise Vinyl oder Methylvinyl und Alkinyl kann beispielsweise Ethinyl oder Propinyl bedeuten.

Für G kommen als Alkalimetallkationen beispielsweise Natrium-, Kalium-und Lithiumkationen, als Erdalkalimetallkationen Magnesium-, Calcium- und Bariumkationen in Betracht. Bevorzugt sind Calcium- und insbesondere Natriumkationen.

Besonders interessant sind Verbindungen der Formel I, worin

a) $R^1$ Halogen, vorzugsweise Fluor oder Chlor, insbesondere Fluor ist;

b) $R^2$ Halogen, vorzugsweise Fluor oder Chlor, insbesondere Fluor ist;

c) $R^3$ Wasserstoff, Fluor oder Chlor, vorzugsweise Wasserstoff ist;

d) $R^4$ Wasserstoff, $R^{10}$CO- oder $R^{11}$NHCO- ist, wobei $R^{10}$ unsubstituiertes oder durch -COOG substituiertes $C_1$-$C_4$-Alkyl bedeutet, worin G für Wasserstoff, ein Alkali- oder Erdalkalimetallkation steht, und $R^{11}$ eine unsubstituierte oder durch Halogen substituierte $C_1$-$C_4$-Alkyl-oder Phenylgruppe bedeutet, wobei die bevorzugte Bedeutung von $R^4$ Wasserstoff ist;

e) $R^5$ Wasserstoff, Halogen, $C_1$-$C_4$-Alkyl oder $C_1$-$C_4$-Haloalkyl, vorzugsweise Wasserstoff, Methyl, $CF_3$, Fluor, Chlor oder Brom, insbesondere Fluor oder Chlor ist;

f) $R^6$ Wasserstoff, Halogen, $C_1$-$C_4$-Alkyl oder $C_1$-$C_4$-Haloalkyl, vorzugsweise Wasserstoff oder $C_1$-$C_2$-Haloalkyl, insbesondere $CF_3$ ist;

g) $R^7$ Wasserstoff, Fluor, Brom oder Chlor, vorzugsweise Wasserstoff, Fluor oder Chlor, insbesondere Chlor ist;

h) $R^8$ Wasserstoff, Fluor, Brom oder Chlor, vorzugsweise Wasserstoff, Fluor oder Chlor, insbesondere Wasserstoff ist;

i) $R^9$ Wasserstoff, Fluor, Brom oder Chlor, vorzugsweise Wasserstoff, Fluor oder Chlor, insbesondere Wasserstoff ist; oder

j) X Schwefel, NH oder Sauerstoff, vorzugsweise Sauerstoff ist.

Wegen ihrer Wirkung als Schädlingsbekämpfungsmittel interessant sind Verbindungen der Formel I, welche zu einer der folgenden Gruppen gehören, worin a) $R^1$ und $R^2$ Halogen, $R^4$ Wasserstoff, $R^{10}CO$- oder $R^{11}NHCO$-, wobei $R^{10}$ für unsubstituiertes oder durch -COOG substituiertes $C_1$-$C_4$-Alkyl steht, $R^5$ und $R^6$ unabhängig voneinander Wasserstoff, Halogen, $C_1$-$C_4$-Alkyl oder $C_1$-$C_4$-Haloalkyl und $R^7$, $R^8$ sowie $R^9$ unabhängig voneinander Wasserstoff, Fluor, Brom oder Chlor bedeuten und $R^3$, $R^{11}$, G und X die in Formel I angegebenen Bedeutungen haben; b) $R^1$ und $R^2$ unabhängig voneinander Fluor oder Chlor, $R^3$ Wasserstoff, Fluor oder Chlor, $R^4$ Wasserstoff, $R^5$ Wasserstoff, Methyl, $CF_3$, Fluor, Chlor oder Brom, $R^6$ Wasserstoff oder $C_1$-$C_2$-Haloalkyl, $R^7$, $R^8$ und $R^9$ unabhängig voneinander Wasserstoff, Fluor oder Chlor und X Schwefel oder Sauerstoff bedeuten; c) $R^1$ und $R^2$ unabhängig voneinander Fluor oder Chlor, $R^3$ Wasserstoff, $R^4$ Wasserstoff, $-COCH_3$, $-COCH_2CH_2COOH$ oder $-CONHCH_3$, $R^5$ Wasserstoff, Fluor, Chlor oder Methyl, $R^6$ $CF_3$ oder $CF_2CCl_2F$, $R^7$ und $R^8$ unabhängig voneinander Wasserstoff oder Chlor, $R^9$ Wasserstoff oder Fluor und X Sauerstoff, Schwefel oder NH bedeuten; d) $R^5$ Fluor oder Chlor, $R^6$ $CF_3$, $R^7$ Chlor, $R^8$ und $R^9$ Wasserstoff und X Sauerstoff bedeuten und $R^1$ bis $R^4$ die in Gruppe c) angegebenen Bedeutungen haben; oder e) $R^1$ und $R^2$ Fluor, $R^3$ und $R^4$ Wasserstoff, $R^5$ Fluor oder Chlor, $R^6$ $CF_3$, $R^7$ Chlor, $R^8$ und $R^9$ Wasserstoff und X Sauerstoff bedeuten.

Besonders hervorzuheben sind die Verbindungen N-[3-(3-Chlor-5-trifluormethyl-pyridyl-2-oxy)-4-chlorphenyl]-N′-[2,6-difluor-3-aminobenzoyl]-harnstoff und N-[3-(3-Chlor-5-trifluormethyl-pyridyl-2-oxy)-4-fluorphenyl]-N′-[2,6-difluor-3-amino-benzoyl]-harnstoff.

Benzoylharnstoffe sind an sich als Wirksubstanzen in Schädlingsbekämpfungsmitteln bekannt, so werden beispielsweise N-[3-(Pyridyl-2-oxy)-phenyl]-N′-benzoylharnstoffe in der EP-0,079,311 (= US-4,677,127) als Insektizide und Akarizide offenbart. Es wurde nun überraschend gefunden, dass die Einführung einer 3-Aminogruppe im Benzoylteil in deutlich günstigeren physiko-chemischen Eigenschaften resultiert (z.B. erhöhte Löslichkeit in protischen Lösungsmitteln) und vor allem zu einer deutlich verbesserten Bioverfügbarkeit nach systemischer Applikation beim Warmblüter führt. Mit den erfindungsgemässen Substanzen erreicht man einerseits hohe Blutplasmawerte bereits unmittelbar nach Applikation und anderseits einen sehr viel rascheren Abbau bzw. rasches Ausscheiden der Aktivsubstanz. Zur Erzielung einer Sofortwirkung bei voller Wirkungshöhe sind die erfindungsgemässen Substanzen wesentlich besser geeignet als die genannten Verbindungen des Standes der Technik.

Die Herstellung von Verbindungen der Formel I lässt sich folgendermassen durchführen:

(a) Verbindungen der Formel I, worin $R^4$ H bedeutet, kann man erhalten durch

(i) Hydrierung einer Verbindung der Formel II

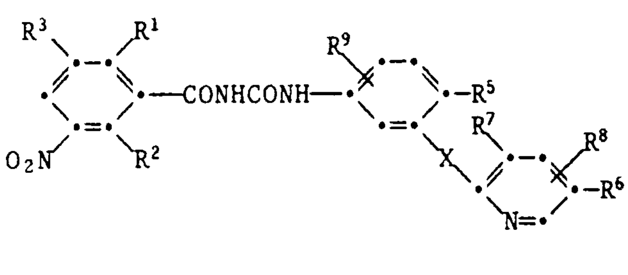

(II)

worin

$R^1$ bis $R^9$ und X die obenstehenden Bedeutungen haben, in Anwesenheit eines geeigneten Katalysators und bei Normaldruck $H_2$ oder

(ii) chemische Reduktion einer Verbindung der Formel II;

(b) Verbindungen der Formel I, worin $R^4$ für $R^{10}CO$- oder $R^{11}NHCO$-steht, können hergestellt werden durch die Umsetzung von Verbindungen der Formel I, worin $R^4$ H bedeutet, mit einer Säure $R^{10}COOH$, einem ihrer Säurehalogenide, ihrem Säureanhydrid oder mit einem Isocyanat $R^{11}NCO$, wobei $R^{10}$ und $R^{11}$ wie unter Formel I definiert sind.

Die erwähnten Verfahren (ai), (aii) und (b) können vorzugsweise in Gegenwart eines organischen Lösungs- oder Verdünnungsmittels durchgeführt werden. Als Lösungs- oder Verdünnungsmittel eignen sich beispielsweise bei den Verfahren (ai) und (aii) Ether (einschliesslich Tetrahydrofuran), Ester und Alkohole, bei Verfahren (b) Ether, unsubstituierte oder durch Substituenten der Gruppe bestehend aus Halogen und $C_1$-$C_4$-Alkyl, vorzugsweise Methyl, substituierte aliphatische oder aromatische Kohlenwasserstoffe, insbesondere Benzol, Toluol, Xylol, Chloroform, Methylenchlorid, Tetrachlorkohlenstoff und Chlorbenzol. Die Verfahren werden im allgemeinen bei einer Temperatur von 0° bis 80°C durchgeführt. Im Fall von Verfahren (ai) und (aii) wird eine Temperatur von 10° bis 50°C bevorzugt, im Fall von Verfahren (b) 20° bis 50°C. Verfahren (b) wird im allgemeinen in Gegenwart einer organischen Base, wie beispielsweise Pyridin, durchgeführt. Als Katalysator für Verfahren (ai) sind beispielsweise 5 % Rh/C oder Raney-Nickel geeignet. Die chemische Reduktion gemäss Verfahren (aii) lässt sich beispielsweise durchführen, indem man eine Verbindung der Formel II mit Sn-(II)-chlorid/HCl behandelt. Im Verfahren (b) ist unter einer Säure, einem Säurehalogenid oder einem Säureanhydrid $R^{10}COOH$, das entsprechende Säurehalogenid oder -anhydrid und unter einem Isocyanat $R^{11}NCO$ zu verstehen.

Die Ausgangsstoffe der Formel II sind neu und stellen ebenfalls einen Gegenstand der vorliegenden Erfindung dar. Besonders interessant sind Verbindungen der Formel II, worin

a) $R^1$ Halogen, vorzugsweise Fluor oder Chlor, insbesondere Fluor ist;

b) $R^2$ Halogen, vorzugsweise Fluor oder Chlor, insbesondere Fluor ist;

c) $R^3$ Wasserstoff, Fluor oder Chlor, vorzugsweise Wasserstoff ist;

d) $R^5$ Wasserstoff, Halogen $C_1$-$C_4$-Alkyl oder $C_1$-$C_4$-Haloalkyl, vorzugsweise Wasserstoff, Methyl, $CF_3$, Fluor, Chlor oder Brom, insbesondere Fluor oder Chlor ist;

e) $R^6$ Wasserstoff, Halogen, $C_1$-$C_4$-Alkyl oder $C_1$-$C_4$-Haloalkyl, vorzugsweise Wasserstoff oder $C_1$-$C_2$-Haloalkyl, insbesondere $CF_3$ ist;

f) $R^7$ Wasserstoff, Fluor, Brom oder Chlor, vorzugsweise Wasserstoff, Fluor oder Chlor, insbesondere Chlor ist;

g) $R^8$ Wasserstoff, Fluor, Brom oder Chlor, vorzugsweise Wasserstoff, Fluor oder Chlor, insbesondere Wasserstoff ist;

h) $R^9$ Wasserstoff, Fluor, Brom oder Chlor, vorzugsweise Wasserstoff, Fluor oder Chlor, insbesondere Wasserstoff ist; oder

i) X Schwefel, NH oder Sauerstoff, vorzugsweise Sauerstoff ist.

Interessant sind diejenigen Verbindungen der Formel II, welche zu einer der nachfolgenden Gruppen gehören, worin a) $R^1$ und $R^2$ Halogen, $R^5$ und $R^6$ unabhängig voneinander Wasserstoff, Halogen, $C_1$-$C_4$-Alkyl oder $C_1$-$C_4$-Haloalkyl und $R^7$, $R^8$ sowie $R^9$ unabhängig voneinander Wasserstoff, Fluor, Brom oder Chlor bedeuten und $R^3$ sowie X die in Formel II angegebenen Bedeutungen haben; b) $R^1$ und $R^2$ unabhängig voneinander Fluor oder Chlor, $R^3$ Wasserstoff, Fluor oder Chlor, $R^5$ Wasserstoff, Methyl, $CF^3$, Fluor, Chlor oder Brom, $R^6$ Wasserstoff oder $C_1$-$C_2$-Haloalkyl, $R^7$, $R^8$ und $R^9$ unabhängig voneinander Wasserstoff, Fluor oder Chlor und X Schwefel, NH oder Sauerstoff bedeuten; c) $R^1$ und $R^2$ unabhängig voneinander Fluor oder Chlor, $R^3$ Wasserstoff, $R^5$ Wasserstoff, Fluor, Chlor oder Methyl, $R^6$ $CF_3$ oder $CF_2CCl_2F$, $R^7$ und $R^8$ unabhängig voneinander Wasserstoff oder Chlor, $R^9$ Wasserstoff oder Fluor und X Sauerstoff, Schwefel oder NH bedeuten; d) $R^5$ Fluor oder Chlor, $R^6$ $CF_3$, $R^7$ Chlor, $R^8$ und $R^9$ Wasserstoff und X Sauerstoff bedeuten und $R^1$, $R^2$ sowie $R^3$ die in c) angegebenen Bedeutungen haben; oder e) $R^1$ und $R^2$ Fluor, $R^3$ Wasserstoff, $R^5$ Fluor oder Chlor, $R^6$ $CF_3$, $R^7$ Chlor, $R^8$ und $R^9$ Wasserstoff und X Sauerstoff bedeuten.

Besonders erwähnenswert sind die Verbindungen N-[3-(3-Chlor-5-trifluormethyl-pyridyl-2-oxy)-4-chlorphenyl]-N'[2,6-difluor-3-nitro-benzoyl]-harnstoff und N-[3-(3-Chlor-5-trifluormethyl-pyridyl-2-oxy)-4-fluorphenyl]-N'[2,6-difluor-3-nitro-benzoyl]-harnstoff.

Die Verbindungen der Formel II können analog bekannter Verfahren hergestellt werden. Solche Verfahren sind unter anderem in den DE-OS 2 123 236, 2 601 780 und 3 240 975 beschrieben.

So können die Verbindungen der Formel II beispielsweise erhalten werden durch Umsetzung

(a) eines Anilins der Formel III

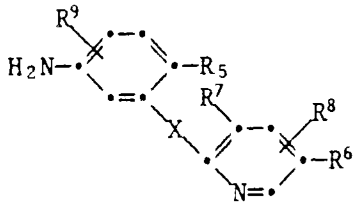

(III)

worin $R^5$ bis $R^9$ und X die obenstehenden Bedeutungen haben, mit einem Benzoylisocyanat der Formel IV

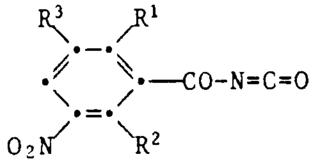

(IV)

worin $R^1$, $R^2$ und $R^3$ die obenstehenden Bedeutungen haben oder
(b) eines Isocyanats der Formel V

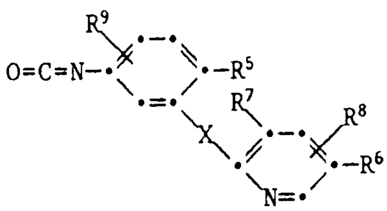

(V)

worin $R^5$ bis $R^9$ und X die obenstehenden Bedeutungen haben, mit einem Benzamid der Formel VI

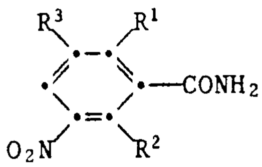

(VI)

worin $R^1$, $R^2$ und $R^3$ die obenstehenden Bedeutungen haben,
oder
(c) eines Anilins der Formel III mit einem Urethan der Formel VII

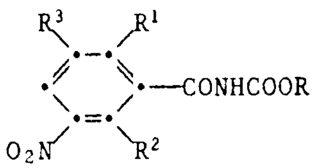

(VII)

worin $R^1$, $R^2$ und $R^3$ die obenstehenden Bedeutungen haben und R $C_1$-$C_5$-Alkyl oder unsubstituiertes oder durch Nitro substituiertes Phenyl bedeutet.

Die erwähnten Verfahren (a), (b) und (c) können vorzugsweise unter normalem Druck und in Gegenwart eines organischen Lösungs- oder Verdünnungsmittels durchgeführt werden. Als Lösungs- oder Verdünnungsmittel eignen sich beispielsweise Ether und etherartige Verbindungen, wie Diethylether, Dipropylether, Dibutylether, Dioxan, Dimethoxyethan und Tetrahydrofuran; N,N-dialkylierte Carbonsäureamide; unsubstituierte oder durch Substituenten der Gruppe bestehend aus Halogen und $C_1$-$C_4$-Alkyl, vorzugsweise Methyl, substituierte aliphatische oder aromatische Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, wie Aceton, Methylethylketon, Methylisopropylketon und Methylisobutylketon. Verfahren (a) wird im allgemeinen bei einer Temperatur von -10° bis +200°C, vorzugsweise zwischen 0° und +100°C, beispielsweise bei Raumtemperatur, gegebenenfalls in Gegenwart einer organischen Base, wie Triethylamin, durchgeführt. Die Durchführung von Verfahren (b) erfolgt bei einer Temperatur von 0° bis +150°C, vorzugsweise beim Siedepunkt des verwendeten Lösungsmittels, und gegebenenfalls in Gegenwart einer organischen Base, wie Pyridin. Für das Verfahren (c), d.h. für die Umsetzung des Urethans der Formel VII mit einem Anilin der Formel III werden Temperaturen zwischen etwa +60° und dem Siedepunkt des jeweiligen Reaktionsgemisches bevorzugt, wobei als Lösungsmittel insbesondere die vorstehend genannten, gegebenenfalls substituierten aromatischen Kohlenwasserstoffe, wie Toluol, Xylole oder Chlorbenzol, verwendet werden.

Die Benzoylisocyanate der Formel IV kann man nach allgemein üblichen Verfahren aus den Benzamiden der Formel VI herstellen, indem man beispielsweise eine Verbindung der Formel VI mit Oxalylchlorid in Gegenwart von Methylenchlorid umsetzt:

$$VI \xrightarrow[CH_2Cl_2]{ClCOCOCl} IV$$

Urethane der Formel VII können analog bekannter Methoden erhalten werden durch Umsetzung eines Benzoylisocyanats der Formel IV mit einem entsprechenden Alkohol R-OH oder durch Umsetzung eines Benzamides der Formel VI in Anwesenheit einer basischen Verbindung mit einem entsprechenden Ester der Chlorameisensäure Cl-COOR, worin R die oben angegebene Bedeutung hat.

Die substituierten Phenylisocyanate der Formel V lassen sich beispielsweise durch Phosgenierung der Aniline der Formel III nach allgemein üblichen Verfahren herstellen.

Bei den Ausgangsstoffen der Formeln IV und VI handelt es sich grösstenteils um neue Verbindungen, die ebenfalls einen Gegenstand der vorliegenden Erfindung bilden. Besonders interessant sind Verbindungen der Formeln IV und VI, worin

a) $R^1$ Halogen, vorzugsweise Fluor oder Chlor, insbesondere Fluor ist; oder

b) $R^2$ Halogen, vorzugsweise Fluor oder Chlor, insbesondere Fluor ist;

Interessant sind diejenigen Verbindungen der Formeln IV und VI, worin $R^1$ und $R^2$ beide Halogen bedeuten. Von besonderem Interesse sind ferner solche Verbindungen der Formeln IV und VI, worin $R^1$ und $R^2$ unabhängig voneinander Fluor oder Chlor bedeuten, insbesondere diejenigen Verbindungen der Formeln IV und VI, worin $R^1$ und $R^2$ Fluor sowie $R^3$ vorzugsweise Wasserstoff bedeuten.

Der Grundvertreter der Formel IV 3-Nitrobenzoylisocyanat ist aus US-3,450,747 bekannt. Ferner wird eine Verbindung der Formel VI, worin $R^3$ für Wasserstoff und $R^1$ und $R^2$ gleichzeitig für Chlor stehen, in Chemical Abstracts Vol. 83, 1975, Abstr. No. 27831, offenbart.

Die Verbindungen der Formel VI lassen sich beispielsweise herstellen durch Nitrierung von Verbindungen der Formel VIII

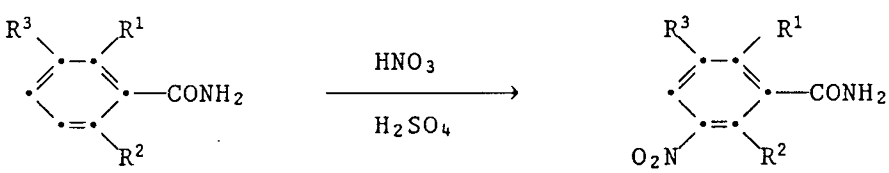


VIII VI

worin $R^1$, $R^2$ und $R^3$ die obenstehenden Bedeutungen haben.

Die Reaktion wird im allgemeinen bei einer Temperatur von -10° bis +100°C, vorzugsweise 0° bis 60°C, durchgeführt.

Die Verbindungen der Formel III sind bekannt oder können analog bekannter Verfahren hergestellt werden, beispielsweise durch Umsetzung eines entsprechenden 2-Chlorpyridinyls mit einem entsprechenden 3-Hydroxyanilin analog dem in der DE-OS 3 240 975 beschriebenen Verfahren.

Die Verbindungen der Formel III mit X = $S(O)_n$ oder NH können hergestellt werden durch Umsetzung einer Verbindung der Formel IX

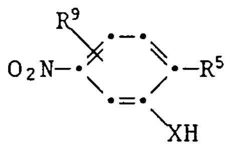

(IX)

worin $R^5$ und $R^9$ die obenstehenden Bedeutungen haben und X für $S(O)_n$ oder NH steht, wobei n 0 bedeutet, mit einer Verbindung der Formel X

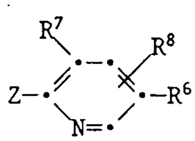

(X)

worin $R^6$ bis $R^8$ die obenstehenden Bedeutungen haben und Z Halogen bedeutet, und anschliessender Hydrierung der so entstandenen Verbindung der Formel XI

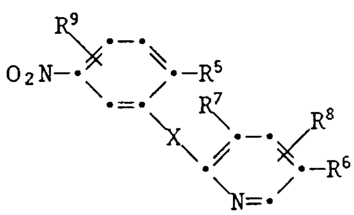

(XI)

worin $R^5$ bis $R^9$ und X die obenstehenden Bedeutungen haben.

Bei den Ausgangsstoffen der Formel XI handelt es sich um neue Verbindungen.

Besonders interessant sind Verbindungen der Formel XI, worin

a) $R^5$ Wasserstoff, Halogen $C_1$-$C_4$-Alkyl oder $C_1$-$C_4$-Haloalkyl, vorzugsweise Wasserstoff, Methyl, $CF_3$, Fluor, Chlor oder Brom, insbesondere Fluor oder Chlor ist;

b) $R^6$ Wasserstoff, Halogen, $C_1$-$C_4$-Alkyl oder $C_1$-$C_4$-Haloalkyl, vorzugsweise Wasserstoff oder $C_1$-$C_2$-Haloalkyl, insbesondere $CF_3$ ist;

c) $R^7$ Wasserstoff, Fluor, Brom oder Chlor, vorzugsweise Wasserstoff, Fluor oder Chlor, insbesondere Chlor ist;

d) $R^8$ Wasserstoff, Fluor, Brom oder Chlor, vorzugsweise Wasserstoff, Fluor oder Chlor, insbesondere Wasserstoff ist;

e) $R^9$ Wasserstoff, Fluor, Brom oder Chlor, vorzugsweise Wasserstoff, Fluor oder Chlor, insbesondere Wasserstoff ist; oder

f) X Schwefel, NH oder Sauerstoff, vorzugsweise Sauerstoff ist oder

welche zu einer der nachfolgenden Gruppe gehören,

worin a) $R^5$ und $R^6$ unabhängig voneinander Wasserstoff, Halogen, $C_1$-$C_4$-Alkyl oder $C_1$-$C_4$-Haloalkyl und $R^7$, $R^8$ sowie $R^9$ unabhängig voneinander Wasserstoff, Fluor, Brom oder Chlor bedeuten und X die in Formel XI angegebene Bedeutung hat; b) $R^5$ Wasserstoff, Methyl, $CF_3$, Fluor, Chlor oder Brom, $R^6$ Wasserstoff oder $C_1$-$C_2$-Haloalkyl, $R^7$, $R^8$ und $R^9$ unabhängig voneinander Waserstoff, Fluor oder Chlor und X Schwefel, NH oder Sauerstoff bedeuten; c) $R^5$ Wasserstoff, Fluor, Chlor oder Methyl, $R^6$ $CF_3$, $R^7$ Wasserstoff oder Chlor, $R^8$ und $R^9$ Wasserstoff sowie X NH bedeuten oder d) $R^5$ Fluor oder Chlor, $R^6$ $CF_3$,

$R^7$ Chlor, $R^8$ und $R^9$ Wasserstoff und X Sauerstoff bedeuten.

Die Herstellung von Verbindungen der Formel XI erfolgt durch die Umsetzung einer Verbindung der Formel IX mit einer Verbindung der Formel X bei einer Temperatur von 50° bis 230°C, vorzugsweise bei 130° bis 190°C. Wenn eine der Verbindungen in flüssiger Form vorliegt, kann die Reaktion ohne Lösungsmittel stattfinden, sonst werden inerte Lösungsmittel wie beispielsweise N,N-Dimethylformamid, Bis(2-methoxyethyl)ether, Tetraoxadodecan oder 2-Ethoxyethanol verwendet.

Die Hydrierung von Verbindungen der Formel XI erfolgt unter $H_2$-Normaldruck und in Anwesenheit inerter Lösungsmittel wie Ether (einschliesslich Tetrahydrofuran), Ester oder Alkohole (auch wässrig). Die Reaktion findet bei einer Temperatur von 0° bis 80°C, vorzugsweise bei 10° bis 50°C statt.

Die Verbindungen der Formel XI, bei denen n 1 oder 2 bedeutet, werden durch Oxidation der Verbindungen der Formel XI, bei denen n O bedeutet, durch an sich bekannte Methoden, wie beispielsweise mit Persäuren (z.B. m-Chlorperbenzoesäure), erhalten.

Die Zwischenprodukte der Formeln II, IV und VI sind Bestandteile der vorliegenden Erfindung. Auf Grund ihrer strukturellen Beschaffenheit sind sie geradezu prädestiniert zur Herstellung von insektizid und akarizid wirksamen Substanzen.

Die Verbindungen der Formel I eignen sich ausgezeichnet zur Bekämpfung von Schädlingen an Tieren sowie Pflanzen und weisen im allgemeinen eine systemische Wirkung auf, vorzugsweise bei oraler Verabreichung oder Injektion bei Haus- und Nutztieren. Sie sind besonders wirksam gegen tierparasitäre Ekto-Parasiten. Zu letzteren zählen Insekten der Ordnungen Homoptera, Heteroptera, Diptera, Thysanoptera, Orthoptera, Anoplura, Siphonaptera, Mallophaga, Thysanura, Isoptera, Psocoptera und Hymenoptera sowie Spinnentiere (Arachnida) der Ordnung Acarina, insbesondere die Familie Ixodidae (Zecken). Die Zecken befallen je nach Art während ihrer Entwicklung von der Larve über die Nymphe zur Imago entweder einen Wirt (einwirtig) oder fallen nach Erreichen des Nymphenstadiums ab, suchen einen neuen Wirt und setzen die Entwicklung fort (zweiwirtig), oder sie parasitieren während jeden Stadiums an einem anderen Tier (dreiwirtig).

Die gute pestizide Wirkung der erfindungsgemässen Verbindungen der Formel I entspricht einer Abtötungsrate (Mortalität) von mindestens 50 bis 60 % der erwähnten Schädlinge. Die insektizide und akarizide Wirkung der Verbindungen der Formel I beruht weniger auf der Abtötung der adulten, parasitären Formen am Körper des Wirtstieres als vielmehr auf einer entwicklungshemmenden Wirkung auf die Larven und Eier, so dass der Lebenszyklus der Parasiten wirkungsvoll unterbrochen ist.

Neben ihrer sehr günstigen Wirkung gegenüber Fliegen, wie beispielsweise *Musca domestica*, und Mückenlarven eignen sich Verbindungen der Formel I vor allem zur Bekämpfung von pflanzenschädigenden Frassinsekten in Zier-und Nutzpflanzungen, insbesondere in Baumwollkulturen (z.B. *Spodoptera littoralis* und *Heliothis virescens*) sowie in Gemüsekulturen (z.B. *Leptinotarsa decemlineata, Pieris brassicae* und *Plutella xylostella*). Hervorzuheben ist auch hier besonders die larvizide und ovizide Wirkung von Verbindungen der Formel I. Werden Verbindungen der Formel I von adulten Insekten mit dem Futter aufgenommen, so ist in vielen Fällen, insbesondere bei Coleopteren, wie beispielsweise *Anthonomus grandis*, eine verminderte Ei-Ablage und/oder reduzierte Schlupfrate festzustellen.

Die Verbindungen der Formel I zeigen auch eine gute anthelminthische Wirkung. Sie sind zur Bekämpfung parasitärer Nematoden beispielsweise der Ordnungen *Rhabditida, Ascaridida, Spirurida, Trichocephalida* oder zur Bekämpfung von Cestoden der Ordnungen *Cyclophyllidae, Pseudophyllidae* oder zur Bekämpfung von Trematoden der Ordnung *Digenea* bei Haus- und Nutztieren wie Rindern, Schafen, Ziegen, Pferden, Schweinen, Katzen, Hunden und Geflügel geeignet. Sie kann den Tieren sowohl als Einzeldosis als auch wiederholt verabreicht werden, wobei die einzelnen Gaben je nach Tierart vorzugsweise zwischen 1 und 500 mg pro kg Körpergewicht betragen. Durch eine protrahierte Verabreichung wird in manchen Fällen eine bessere Wirkung erzielt oder man kann mit geringeren Gesamtdosen auskommen.

Die anthelmintische Wirkung der Verbindungen der Formel I beruht weniger auf der Abtötung der Adulten, parasitären Formen im Körper des Wirtstieres als vielmehr auf einer entwicklungshemmenden Wirkung auf die Larven und auf die mit dem Kot ausgeschiedenen Eier, so dass der Lebenszyklus des Parasiten wirkungsvoll unterbrochen wird.

Die Verbindungen der Formel I sind auch wirksam gegen Pflanzen-Nematoden der Gattungen *Meloidogyne, Heterodera, Pratylenchus, Ditylenchus, Radopholus, Rhizoglyphus* und andere.

Die Verbindungen der Formel I werden in unveränderter Form oder vorzugsweise zusammen mit den in der Formulierungstechnik üblichen Hilfsmitteln eingesetzt und werden daher beispielsweise zu Emulsionskonzentraten, verdünnbaren oder direkt versprühbaren Lösungen, verdünnten Emulsionen, Spritzpulvern, löslichen Pulvern, Stäubemitteln, Granulaten, auch Verkapselungen in beispielsweise polymeren Stoffen, in bekannter Weise verarbeitet. Die Anwendungsverfahren wie Versprühen, Vernebeln, Verstäuben, Verstreuen

oder Giessen und vor allem die orale Verabreichung sowie Injizieren werden wie die Art der Mittel den angestrebten Zielen und den gegebenen Verhältnissen entsprechend gewählt.

Die Verbindungen der Formel I werden bei Warmblütern in Aufwandmengen von 0,01 bis 100 mg/kg Körpergewicht dosiert. Ueber geschlossenen Kultur-Anbauflächen werden sie in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, beispielsweise durch inniges Vermischen und/oder Vermahlen der Wirkstoffe mit Streckmitteln, wie mit Lösungsmitteln oder festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie Bimsstein, Ziegelbruch, Sepiolit oder Bentonit, als nicht sorptive Trägermaterialien beispielsweise Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$ bis $C_{22}$), wie die Na-oder K-Salze der Oel- oder Stearinsäure, oder von natürlichen Fettsäuregemischen, die beispielsweise aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, beispielsweise das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8 bis 22 Kohlenstoff-Atomen. Alkylarylsulfonate sind beispielsweise die Na-, Ca-oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate, wie Salze des Phosphorsäureesters eines p-Nonylphenol-(4-14)-Ethylenoxyd-Adduktes, oder Phospholipide in Frage.

Die in der Formulierungstechnik gebräuchlichen Tenside sind u.a. in folgenden Publikationen beschrieben:

"1986 International McCutcheon's Emulsifiers and Detergents" The Manufacturing Confectioner Publishing Co.,

Glen Rock, New Jersey, USA und

Stache, H., Tensid-Taschenbuch, Hanser-Verlag München, Wien, 1981.

Die pestiziden Zubereitungen enthalten in der Regel 0,01 bis 95 %, insbesondere 0,1 bis 80 % Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1 bis 10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

H-1: N-[3-(3-Chlor-5-trifluormethyl-pyridyl-2-oxy)-4-chlorphenyl]-N'-[2,6-dichlor-3-amino-benzoyl]-harnstoff

a) 2,6-Dichlor-3-nitro-benzoesäureamid

Zu einer Lösung von 30,4 g 2,6-Dichlorbenzoesäureamid in 60 ml conc. $H_2SO_4$ (d = 1.83) wird bei Raumtemperatur innert 30 Minuten eine Lösung von 12 g rauchender $HNO_3$ (d = 1,52) in 11 ml conc. $H_2SO_4$ gegeben. Nach 2 Stunden Rühren bei Raumtemperatur wird das Reaktionsgemisch auf Eis/$H_2O$ gegeben und mit 30 % NaOH auf pH ~ 6 gestellt, die ausfallenden Kristalle werden abfiltriert. Man erhält 28,7 g 2,6-Dichlor-3-nitrobenzoesäureamid (Smp 163-165 °C).

b) 2,6-Dichlor-3-nitro-benzoylisocyanat

Zu einer Suspension von 7,68 g 2,6-Dichlor-3-nitro-benzoesäureamidin 570 ml Methylenchlorid wird 3,5 ml Oxalylchlorid innert 30 Minuten bei Raumtemperatur zugetropft. Anschliessend wird 18 Stunden auf Rückflusstemperatur erhitzt und dann das Lösungsmittel im Vakuum abdestilliert. Man erhält 8,6 g rohes 2,6-Dichlor-3-nitro-benzoylisocyanat, das direkt für die weiteren Umsetzungen verwendet werden kann.

c) N-[3-(3-Chlor-5-trifluormethyl-pyridyl-2-oxy)-4-chlorphenyl]-N'-[2,6-dichlor-3-nitro-benzoyl]-harnstoff

20,8 g 2,6-Dichlor-3-nitro-benzoylisocyanat, gelöst in 320 ml Methylenchlorid, werden bei Raumtemperatur mit 23,3 g 3-(3-Chlor-5-trifluormethyl-pyridyl-2-oxy)-4-chloranilin, gelöst in 50 ml Methylenchlorid, versetzt und 19 Stunden gerührt. Anschliessend wird das Reaktionsgemisch mit 200 ml Toluol versetzt und etwa 80 % des Lösungsmittels in einem Rotationsverdampfer entfernt. Der entstandene Niederschlag wird abfiltriert, mit wenig kaltem Toluol und Hexan gewaschen und im Vakuum getrocknet. Man erhält N-[3-(3-Chlor-5-trifluormethyl-pyridyl-2-oxy)-4-chlorphenyl]-N'-[2,6-dichlor-3-nitro-benzoyl]-harnstoff als weisses, kristallines Pulver (Smp. 201-203 °C).

d) N-[3-(3-Chlor-5-trifluormethyl-pyridyl-2-oxy)-4-chlorphenyl]-N'-[2,6-dichlor-3-amino-benzoyl]-harnstoff

3,0 g N-[3-(3-Chlor-5-trifluormethyl-pyridyl-2-oxy)-4-chlorphenyl]-N'-[2,6-dichlor-3-nitro-benzoyl]-harnstoff werden in 60 ml Tetrahydrofuran gelöst und während 6 Stunden bei Raumtemperatur in Gegenwart von 1 g 5 % Rhodium-Kohle hydriert. Das Reaktionsgemisch wird anschliessend filtriert und das Lösungsmittel abdestilliert. Das erhaltene Rohprodukt wird aus Diethylether/Hexan umkristallisiert. Man erhält 2 g N-[3-(3-Chlor-5-trifluormethyl-pyridyl-2-oxy)-4-chlorphenyl]-N'-[2,6-dichlor-3-amino-benzoyl]-harnstoff als farblose Kristalle (Smp. 207-209 °C).

H-2: N-[3-(3-Chlor-5-trifluormethyl-pyridyl-2-oxy)-4-chlorphenyl]-N'-[2,6-difluor-3-(N-acetyl-amino)-benzoyl]-harnstoff

a) 2,6-Difluor-3-nitro-benzoesäureamid

Analog Vorschrift H-1a) erhält man aus 23,1 g 2,6-Difluor-benzoesäureamid 22,6 g 2,6-Difluor-3-nitrobenzoesäureamid (Smp. 134-135 °C).

b) 2,6-Difluor-3-nitro-benzoylisocyanat

2,6-Difluor-3-nitro-benzoylisocyanat wird analog Vorschrift H-1b) aus 2,6-Difluor-3-nitro-benzoesäureamid hergestellt.

c) N-[3-(3-Chlor-5-trifluormethyl-pyridyl-2-oxy)-4-chlorphenyl]-N′-[2,6-difluor-3-nitro-benzoyl]-harnstoff

Zu einer Lösung von 10,0 g 2,6-Difluor-3-nitro-benzoylisocyanat in 190 ml Ethylenchlorid wird eine Lösung von 12,9 g 3-(3-Chlor-5-trifluormethylpyridyl-2-oxy)-4-chloranilin in 30 ml Ethylenchlorid gegeben und das erhaltene Reaktionsgemisch 5 Stunden bei Raumtemperatur gerührt. Anschliessend wird das Lösungsmittel entfernt, der Rückstand mit 70 ml Toluol verrührt und der entstandene Niederschlag abfiltriert. Man erhält 15,1 g N-[3-(3-Chlor-5-trifluormethyl-pyridyl-2-oxy)-4-chlorphenyl]-N′-[2,6-difluor-3-nitro-benzoyl]-harnstoff als farblose Kristalle (Smp. 186-187 °C).

d) N-[3-(3-Chlor-5-trifluormethyl-pyridyl-2-oxy)-4-chlorphenyl]-N′-[2,6-difluor-3-amino-benzoyl]-harnstoff

Analog Vorschrift H-1d) erhält man aus 15 g N-[3-(3-Chlor-5-trifluormethyl-pyridyl-2-oxy)-4-chlorphenyl]-N′-[2,6-difluor-3-nitro-benzoyl]-harnstoff 10,6 g N-[3-(3-Chlor-5-trifluormethyl-pyridyl-2-oxy)-4-chlorphenyl]-N′-[2,6-difluor-3-amino-benzoyl]-harnstoff (Smp. 181-182 °C).

e) N-[3-(3-Chlor-5-trifluormethyl-pyridyl-2-oxy)-4-chlorphenyl]-N′-[2,6-difluor-3-(N-acetyl-amino)-benzoyl]-harnstoff

Zu einer Lösung von 2,0 g N-[3-(3-Chlor-5-trifluormethyl-pyridyl-2-oxy)-4-chlorphenyl]-N′-[2,6-difluor-3-amino-benzoyl] -harnstoff und 0,31 ml Pyridin in 38 ml Methylenchlorid werden 0,33 ml Acetylchlorid gegeben und 3 Stunden bei 0 °C gerührt. Der entstandene Niederschlag wird abfiltriert, mit 1 N HCl, Wasser und Methylenchlorid gewaschen und im Vakuum getrocknet. Man erhält 1,1 g N-[3-(3-Chlor-5-trifluormethyl-pyridyl-2-oxy)-4-chlorphenyl]-N′-[2,6-difluor-3-(N-acetyl-amino)-benzoyl]-harnstoff (Smp. 209-211 °C).

H-3: N-[3-(3-Chlor-5-trifluormethyl-pyridyl-2-oxy)-4-fluorphenyl]-N′-[2,6-difluor-3-amino-benzoyl]-harnstoff

a) N-[3-(3-Chlor-5-trifluormethyl-pyridyl-2-oxy)-4-fluorphenyl]-N′-[2,6-difluor-3-nitro-benzoyl]-harnstoff

8,6 g 2,6-Difluor-3-nitro-benzoylisocyanat (Herstellung siehe H-2b), gelöst in 300 ml Methylenchlorid, werden bei Raumtemperatur mit 9,7 g 3-(3-Chlor-5-trifluormethyl-pyridyl-2-oxy)-4-fluoranilin, gelöst in 10 ml Methylenchlorid, versetzt und 3 Stunden bei Raumtemperatur gerührt. Das Reaktionsgemisch wird dann mit 300 ml Toluol versetzt und etwa 80 % des Lösungsmittels im Vakuum abdestilliert. Der erhaltene Niederschlag wird abfiltriert und mit wenig Toluol und Hexan gewaschen. Man erhält 11,9 g N-[3-(3-Chlor-5-trifluormethyl-pyridyl-2-oxy)-4-fluorphenyl]-N′-[2,6-difluor-3-nitro-benzoyl]-harnstoff als farblose Kristalle (Smp. 183-185 °C).

b) N-[3-(3-Chlor-5-trifluormethyl-pyridyl-2-oxy)-4-fluorphenyl]- N′-[2,6-difluor-3-amino-benzoyl]-harnstoff

8,8 g N-[3-(3-Chlor-5-trifluormethyl-pyridyl-2-oxy)-4-fluorphenyl]-N′-[2,6-difluor-3-nitro-benzoyl]-harnstoff werden in 180 ml Tetrahydrofuran gelöst und 17 Stunden bei Raumtemperatur in Gegenwart von 15 g Raney-Nickel hydriert. Das Reaktionsgemisch wird anschliessend filtriert und das Lösungsmittel im Vakuum abdestilliert. Das erhaltene Rohprodukt wird aus Diethylether umkristallisiert. Man erhält 6,7 g N-[3-(3-Chlor-5-trifluormethyl-pyridyl-2-oxy)-4-fluorphenyl]-N′-[2,6-difluor-3-aminobenzoyl]-harnstoff als farblose Kristalle (Smp. 174-175 °C).

H-4) N-[3-(3-Chlor-5-trifluormethyl-pyridyl-2-oxy)-4-chlorphenyl]-N′-[2,6-difluor-3-(N-carboxypropionylamino)-benzoyl]-harnstoff

Eine Lösung von 2,0 g N-[3-(3-Chlor-5-trifluormethyl-pyridyl-2-oxy)-4-chlorphenyl]-N′-[2,6-difluor-3-amino-benzoyl]-harnstoff (Herstellung siehe H-2d), 0,46 g Bernsteinsäureanhydrid und 14 ml Tetrahydrofuran wird 2 Tage bei Raumtemperatur gerührt. Anschliessend wird das Lösungsmittel im Vakuum abdestilliert und der Rückstand mit 50 ml Methylenchlorid verrührt. Der erhaltene Niederschlag wird abfiltriert und mit Diethylether gewaschen. Man erhält N-[3-(3-Chlor-5-trifluormethyl-pyridyl-2-oxy)-4-chlorphenyl]-N′-[2,6-difluor-3-(N-carboxypropionylamino)-benzoyl]-harnstoff als weisses Pulver (Smp. 223-225 °C).

H-5) N-[3-(3-Chlor-5-trifluormethyl-pyridyl-2-oxy)-4-chlorphenyl]-N′-[2,6-difluor-3-(N-methylcarbamoyl-amino)-benzoyl]-harnstoff

Eine Lösung von 3,84 g N-[3-(3-Chlor-5-trifluormethyl-pyridyl-2-oxy)-4-chlorphenyl]-N′-[2,6-difluor-3-amino-benzoyl]-harnstoff (Herstellung siehe H-2d), 0,27 ml Methylisocyanat und 0,31 ml Pyridin in 50 ml Methylenchlorid wird 5 Tage bei Raumtemperatur gerührt. Der entstandene Niederschlag wird abfiltriert, mit etwas Methylenchlorid und Diethylether nachgewaschen und im Vakuum getrocknet. Man erhält 1,47 g N-[3-(3-Chlor-5-trifluormethyl-pyridyl-2-oxy)-4-chlorphenyl]-N′-[2,6-difluor-3-(N-methylcarbamoyl-amino)-benzoyl]-harnstoff (Smp. 209-211 °C).

H-6: N-(5-Trifluormethyl-2-pyridyl)-2-methyl-5-aminoanilin

a) N-(5-Trifluormethyl-2-pyridyl)-2-methyl-5-nitroanilin

3,04 g (0.02 mmol) 2-Amino-4-nitrotoluol und 7,26 g (0.04 mol) 2-Chlor-5-trifluormethylpyridin werden in der Schmelze während 5 Stunden bei 160 °C gerührt. Nach dem Abkühlen wird 200 ml Wasser dazugegeben und das Produkt in Methylenchlorid aufgenommen. Das Lösungsmittel wird abgedampft und der Rückstand mittels Säulenchromatographie gereinigt (Silicagel, Methylenchlorid:Hexan = 2:1). Man erhält 4,4 g N-(5-Trifluormethyl-2-pyridyl)-2-methyl-5-nitroanilin (Smp. 103-105 °C).

b) N-(5-Trifluormethyl-2-pyridyl)-2-methyl-5-aminoanilin

4,2 g der in a) erhaltenen Substanz werden in 80 ml Tetrahydrofuran bei 30-35 °C mit 4 g Raney-Nickel unter Normaldruck hydriert. Der Katalysator wird abfiltriert und das Lösungsmittel eingedampft. Der Rückstand wird mit Hexan aufgeschlämmt, die erhaltenen Kristalle werden filtriert und getrocknet. Man erhält 3,4 g N-(5-Trifluormethyl-2-pyridyl)-2-methyl-5-aminoanilin als weisses Pulver (Smp. 113-115 °C).

N-(5-Trifluormethyl-2-pyridyl)-2-methyl-5-aminoanilin kann in den Beispielen H-1 und H-2 anstelle der in c) eingesetzten Pyridyloxyaniline zur Herstellung von Verbindungen eingesetzt werden, in denen X für NH steht.

H-7: N-(3-Chlor-5-trifluormethyl-2-pyridyl)-2-chlor-5-aminoanilin

a) N-(3-Chlor-5-trifluormethyl-2-pyridyl)-2-chlor-5-nitroanilin

3,4 g (0.02 mol) 2-Chlor-5-nitroanilin, 8,6 g (0.04 mol) 2,3-Dichlor-5-trifluormethylpyridin und 400 mg Kupferpulver werden in einem kleinen Bombenrohr (25 ml) über Nacht bei 230 °C gehalten. Nach dem Abkühlen wird das Rohprodukt mittels Säulenchromatographie gereinigt (Silicagel, Petrolether:Methylenchlorid = 6:1). Man erhält 1,6 g N-(3-Chlor-5-trifluormethyl-2-pyridyl)-2-chlor-5-nitroanilin als gelbes Pulver (Smp. 122-124 °C).

b) N-(3-Chlor-5-trifluormethyl-2-pyridyl)-2-chlor-5-aminoanilin

1,4 g der in a) erhaltenen Substanz werden in 80 ml Tetrahydrofuran bei 30-35 °C mit 2 g Rhodium (5 % auf Kohle) unter Normaldruck hydriert. Der Katalysator wird abfiltriert und das Lösungsmittel eingedampft. Der Rückstand wird mit Petrolether aufgeschlämmt. Die erhaltenen Kristalle werden filtriert und getrocknet. Man erhält 1,1 g N-(3-Chlor-5-trifluormethyl-2-pyridyl)-2-chlor-5-aminoanilin als weisses Pulver (Smp. 111-113 °C).

N-(3-Chlor-5-trifluormethyl-2-pyridyl)-2-chlor-5-aminoanilin kann in den Beispielen H-1 und H-2 anstelle der in c) eingesetzten Pyridyloxyaniline zur Herstellung von Verbindungen eingesetzt werden, in denen X für NH steht.

Analog vorstehend beschriebener Verfahren werden die folgenden, in den Tabellen zusammen mit den Verbindungen der vorgängigen Herstellungsbeispiele aufgeführten Verbindungen hergestellt:

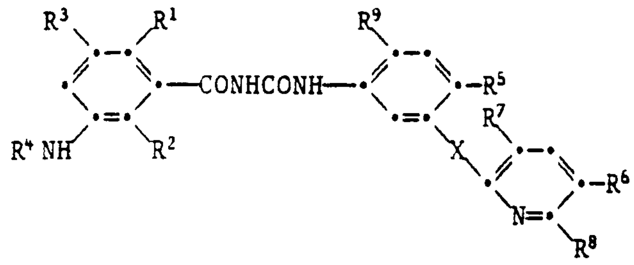

(I)

Tabelle 1

| Nr. | $R^1$ | $R^2$ | $R^3$ | $R^4$ | $R^5$ | $R^6$ | $R^7$ | $R^8$ | $R^9$ | X | Smp.(°C) |
|---|---|---|---|---|---|---|---|---|---|---|---|
| 1.1 | F | F | H | H | Cl | $CF_3$ | Cl | H | H | O | 181-182 |
| 1.2 | Cl | Cl | H | H | Cl | $CF_3$ | Cl | H | H | O | 207-209 |
| 1.3 | F | F | H | $COCH_3$ | Cl | $CF_3$ | Cl | H | H | O | 209-211 |
| 1.4 | F | F | H | $CONHCH_3$ | Cl | $CF_3$ | Cl | H | H | O | 209-211 |
| 1.5 | F | F | H | $CO(CH_2)_2COOH$ | Cl | $CF_3$ | Cl | H | H | O | 223-225 |
| 1.6 | F | F | H | H | F | $CF_3$ | Cl | H | H | O | 174-175 |
| 1.7 | F | F | H | H | Cl | $CF_3$ | Cl | Cl | H | O | 208-210 |
| 1.8 | F | F | H | H | Cl | $CF_3$ | H | H | F | O | 189-191 |
| 1.9 | F | F | H | H | Cl | $CF_2CCl_2F$ | Cl | H | H | O | 187-191 |
| 1.10 | F | F | H | H | $CH_3$ | $CF_3$ | Cl | H | H | NH | 202-204 |
| 1.11 | F | F | H | H | Cl | $CF_3$ | Cl | H | H | NH | 214-216 |
| 1.12 | F | F | H | H | H | $CF_3$ | Cl | H | H | S | 193-194 |
| 1.13 | F | F | H | H | H | $CF_3$ | H | H | H | S | |

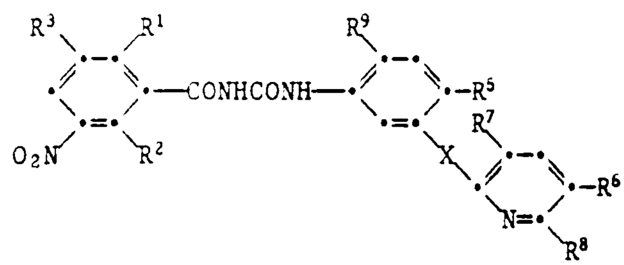

(II)

Tabelle 2

| Nr. | $R^1$ | $R^2$ | $R^3$ | $R^5$ | $R^6$ | $R^7$ | $R^8$ | $R^9$ | X | Smp.(°C) |
|---|---|---|---|---|---|---|---|---|---|---|
| 2.1 | F | F | H | Cl | $CF_3$ | Cl | H | H | O | 186-187 |
| 2.2 | Cl | Cl | H | Cl | $CF_3$ | Cl | H | H | O | 201-203 |
| 2.3 | F | F | H | F | $CF_3$ | Cl | H | H | O | 183-185 |
| 2.4 | F | F | H | Cl | $CF_3$ | Cl | Cl | H | O | 188 (Zersetzung |
| 2.5 | F | F | H | Cl | $CF_3$ | H | H | F | O | 195-201 |
| 2.6 | F | F | H | Cl | $CF_2CCl_2F$ | Cl | H | H | O | 201-205 |
| 2.7 | F | F | H | $CH_3$ | $CF_3$ | Cl | H | H | NH | 212-214 |
| 2.8 | F | F | H | Cl | $CF_3$ | Cl | H | H | NH | 229-231 |
| 2.9 | F | F | H | H | $CF_3$ | Cl | H | H | S | 205-207 |
| 2.10 | F | F | H | H | $CF_3$ | H | H | H | S | |

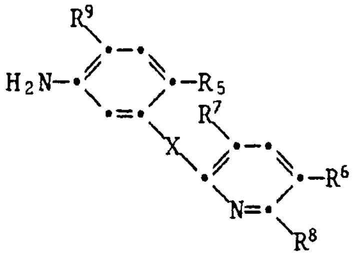

(III)

Tabelle 3

| Nr. | $R^5$ | $R^6$ | $R^7$ | $R^8$ | $R^9$ | X | Smp.(°C) |
|---|---|---|---|---|---|---|---|
| 3.1 | H | $CF_3$ | H | H | H | S | |
| 3.2 | H | $CF_3$ | Cl | H | H | S | 115-117 |
| 3.3 | H | $CF_3$ | H | H | H | NH | 106-109 |
| 3.4 | H | $CF_3$ | Cl | H | H | NH | 102-104 |
| 3.5 | F | $CF_3$ | H | H | H | NH | 105-107 |
| 3.6 | F | $CF_3$ | Cl | H | H | NH | 105-107 |
| 3.7 | Cl | $CF_3$ | H | H | H | NH | 117-119 |
| 3.8 | Cl | $CF_3$ | Cl | H | H | NH | 111-113 |
| 3.9 | $CH_3$ | $CF_3$ | H | H | H | NH | 113-115 |
| 3.10 | $CH_3$ | $CF_3$ | Cl | H | H | NH | 160-162 |

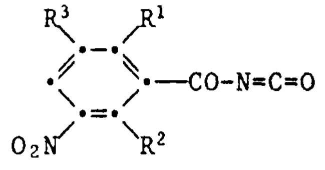

(IV)

Tabelle 4

| Nr. | $R^1$ | $R^2$ | $R^3$ |
|---|---|---|---|
| 4.1 | Cl | Cl | H |
| 4.2 | F | F | H |

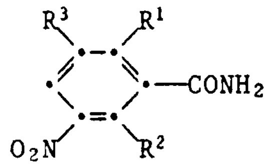

(VI)

Tabelle 5

| Nr. | $R^1$ | $R^2$ | $R^3$ | Smp.(°C) |
|---|---|---|---|---|
| 5.1 | F | F | H | 134-135 |
| 5.2 | Cl | Cl | H | 163-165 |

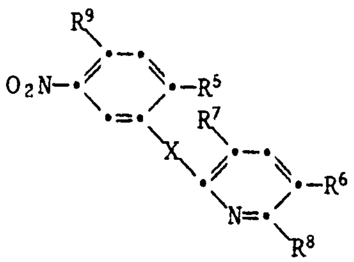

(XI)

Tabelle 6

| Nr. | $R^5$ | $R^6$ | $R^7$ | $R^8$ | $R^9$ | X | Smp.(°C) |
|---|---|---|---|---|---|---|---|
| 6.1 | H | $CF_3$ | H | H | H | NH | 137-138 |
| 6.2 | H | $CF_3$ | Cl | H | H | NH | 119-121 |
| 6.3 | F | $CF_3$ | H | H | H | NH | 84- 85 |
| 6.4 | F | $CF_3$ | Cl | H | H | NH | 112-114 |
| 6.5 | Cl | $CF_3$ | H | H | H | NH | 157-159 |
| 6.6 | Cl | $CF_3$ | Cl | H | H | NH | 122-124 |
| 6.7 | $CH_3$ | $CF_3$ | H | H | H | NH | 93- 97 |
| 6.8 | $CH_3$ | $CF_3$ | Cl | H | H | NH | 140-142 |
| 6.9 | H | $CF_3$ | Cl | H | H | S | 115-117 |

Formulierungsbeispiele für feste Wirkstoffe der Formel I (% = Gewichtsprozent)

| F-1) Spritzpulver | | | |
|---|---|---|---|
| | a) | b) | c) |
| Wirkstoff gemäss Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7 bis 8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält ein Spritzpulver, das sich mit Wasser zu Suspensionen jeder gewünschten Konzentration verdünnen lässt. Das Pulver kann mit dem Futter appliziert werden.

| F-2) Emulsions-Konzentrat | |
|---|---|
| Wirkstoff gemäss Tabelle 1 | 10 % |
| Octylphenolpolyethylenglykolether (4 bis 5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F-3) Stäubemittel | | |
|---|---|---|
| | a) | b) |
| Wirkstoff gemäss Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird. Stäubemittel können direkt appliziert oder dem Futter beigegeben werden.

| F-4) Extruder-Granulat | |
|---|---|
| Wirkstoff gemäss Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet. Solche Granulate eignen sich auch als Futter-Zusatzstoffe.

| F-5) Umhüllungs-Granulat | |
|---|---|
| Wirkstoff gemäss Tabelle 1 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate. Solche Granulate eignen sich auch als Futter-Zusatzstoffe.

| F-6) Suspensions-Konzentrat | |
|---|---|
| Wirkstoff gemäss Tabelle 1 | 40 % |
| Ethylenglykol | 10 % |
| Nonylphenolpolyethylenglykolether (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensionskonzentrat, aus welchem durch Verdünnen mit Wasser Suspensionen jeder gewünschten Konzentration hergestellt werden können. Suspensionskonzentrate können am Tier auch oral appliziert werden ("Drench").

<u>F-7) Komprimate</u>

| | | |
|---|---|---|
| I | Wirkstoff gemäss Tabelle 1 | 33,0 % |
| | Methylcellulose | 0,8 % |
| | Kieselsäure (hochdispers) | 0,8 % |
| | Maisstärke | 8,4 % |
| II | Milchzucker (kristallin) | 22,5 % |
| | Maisstärke | 17,0 % |
| | mikrokristalline Cellulose | 16,5 % |
| | Magnesiumstearat | 1,0 % |

Die Hilfsstoffe der Phase I werden mit dem Wirkstoff unter Zusatz von 16,5 Teilen Wasser granuliert und dann mit den Hilfsstoffen der Phase II vermischt. Die Mischung wird zu Tabletten oder Boli verpresst und getrocknet.

F-8) Injizierbare Lösungen

| | | | |
|---|---|---|---|
| a) | I | Wirkstoff gemäss Tabelle 1 | 5 g |
| | II | polyoxyethyliertes Ricinusöl, hydriert (EO 40) | 30 g |
| | III | N-Methylpyrrolidon | ad 100 ml |
| b) | I | Wirkstoff gemäss Tabelle 1 | 5 g |
| | II | polyoxyethyliertes Ricinusöl, hydriert (EO 40) | 15 g |
| | III | N-Methylpyrrolidon | ad 100 ml |
| c) | I | Wirkstoff gemäss Tabelle 1 | 5 g |
| | II | polyoxyethyliertes Ricinusöl, hydriert (EO 40) | 20 g |
| | | Benzylalkohol | 10 g |
| | | DMSO (Dimethylsulfoxid) | 20 g |
| | III | N-Methylpyrrolidon | ad 100 ml |

Der Wirkstoff wird mit einem Teil der Komponente III versetzt und unter Rühren und gegebenenfalls leichtem Erwärmen vollständig gelöst. Nach Zusatz von II und inniger Durchmischung wird mit III auf das Sollvolumen aufgefüllt. Die fertige Mischung wird sterilfiltriert (Membranfiltration 0,22 µm) und bei 121 °C 15 Minuten sterilisiert.

In allen Beispielen kann ein Teil des Wirkstoffs gemäss Tabelle 1 durch andere Anthelmintika, Insektizide oder Akarizide ersetzt werden.

Biologische Beispiele

B-1) Versuche an mit *Haemonchus contortus* infizierten Schafen

a) Wirkung auf die Larvenentwicklung im Wirt

An Schafe wird mit Hilfe einer Schlundsonde während 10 Tagen täglich eine bestimmte Menge der zu prüfenden Aktivsubstanz verabreicht. Die Dosierungen variieren zwischen 0.01 und 100 mg pro kg Lebendgewicht. Die Infektion mit infektiösen *H. contortus* Larven erfolgt am 2. Tag nach Behandlungsbeginn.

Die Evaluierung der Wirkung erfolgt durch Auszählung der Eier in Kotproben, die 21 bis 35 Tage nach der Infektion den Schafen rectal entnommen werden. Die Wirkung zeigt sich darin, dass die Kotproben der mit der Verbindung der Formel I behandelten Schafe keine Wurmeier enthalten im Gegensatz zu den Kontrolltieren. Das Fehlen von Wurmeiern zeigt, dass *H. contortus* sich nicht normal entwickeln kann.

b) Wirkung auf *H. contortus* Eier

Massiv mit adulten *H. contortus* infizierten Schafen wird während 14 Tagen täglich mit Hilfe einer Schlundsonde die Verbindung der Formel I in einer Dosierung von 10 mg pro kg Lebendgewicht verabreicht.

Die Evaluierung der Wirkung erfolgt durch Auszählen der infektiösen Larven in inkubierten Kotproben, die zwischen dem 3. und 21. Tag nach Behandlungsbeginn den Schafen rectal entnommen werden. Die

Wirkung zeigt sich darin, dass aus zwischen dem 3. und 14. Behandlungstag abgelegten Eiern keine Larven entstehen, wohl aber aus Eiern unbehandelter Kontrolltiere und aus Eiern, die nach Behandlungsabbruch abgelegt werden.

B-2) Wirkung gegen Eier des Leberegels *Fasciola hepatica*

*F. hepatica* Eier werden in wässrigem Milieu Konzentrationen von 7.5, 75 und 750 ppm der Verbindung der Formel I ausgesetzt und bei Raumtemperatur über 15 Tage im Dunkeln aufbewahrt.

Mikroskopische Untersuchung der Eier nach 15 Tagen ergibt, dass sich bei den beiden höheren Konzentrationen keine Miracidien entwickeln und die Eier total verformt sind.

In den Versuchen B-1a) und b) sowie B-2) zeigen die Verbindungen der Formel I ausgeprägte entwicklungshemmende Wirkung auf die Eier, Larven bzw. Miracidien (Hemmung 95 bis 100 % gegenüber unbehandeltem Wirt).

B-3) Reproduktionshemmende Wirkung bei Zecken

Frische, vollgesogene Weibchen der Zeckenart *Boophilus microplus* werden in Reihen zu 10 Tieren dorsal auf PVC-Platten aufgeklebt und mit einem Wattebausch überdeckt. Danach wird jede Reihe mit 10 ml der wässrigen Testlösung übergossen. Eine Stunde später wird der Wattebausch entfernt, die Zecken werden über Nacht bei 24 °C getrocknet. Nach dem Trocknen werden die Zecken bei 28 °C und 80 % Luftfeuchtigkeit für 4 Wochen bis zum Ende der Eiablage und Beginn des Larvenschlupfes gehalten.

Jede Testsubstanz wird bei 5 Konzentrationen von 1000 bis 62 ppm (Verdünnungsfaktor 2) getestet. Die akarizide Wirkung zeigt sich entweder beim Weibchen als Mortalität oder Sterilität oder im Eigelege durch Blockieren der Embryogenese oder des Schlüpfaktes. Alle Substanzen werden gegen zwei verschiedene Zeckenstämme, den normal sensiblen Stamm YEERONGPILLY und den OP-resistenten Stamm BIARRA geprüft.

Die Aktivität einer Substanz wird auf Grund der tiefsten noch annähernd voll aktiven Konzentration bewertet (IR 90 ~ 90 % Inhibition der Reproduktion).

Verbindungen der Formel I, wie beispielsweise Verbindungen Nr. 1.1, 1.6, 1.7, 1.9 und 1.12, zeigen in diesem Test eine mehr als 90 % reproduktionshemmende Wirkung bei Konzentrationen von 62 ppm.

B-4) Reproduktions-Beeinflussung von *Anthonomus grandis*

Adulte *Anthonomus grandis*, die nach dem Verlassen der letzten Puppenhülle nicht älter als 24 Stunden sind, werden in Gruppen zu jeweils 25 Käfern in Käfige mit Gitterwänden überführt. Die mit den Käfern besetzten Käfige werden sodann während 5 bis 10 Sekunden in eine acetonische Lösung, die 800 ppm des zu prüfenden Wirkstoffes enthält, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I zeigen eine gute reproduktionsreduzierende Wirkung im obigen Test.

B-5) Wirkung geben *Aëdes aegypti*

Auf die Oberfläche von 150 ml Wasser in einem Anzuchtbehälter wird so viel einer 0,1%igen acetonischen Lösung des Wirkstoffes pipettiert, dass eine Konzentration von 12,5 ppm erhalten wird. Nach Verdunsten des Acetons wird der Behälter mit 30 bis 40 2tägigen *Aëdes*-Larven beschickt. Nach 2 und 7 Tagen wird die Mortalität geprüft.

Verbindungen gemäss Formel 1 zeigen gute Wirkung in diesem Test.

B-6) Wirkung gegen Schmeissfliegen

Frisch abgelegte Eier der Schmeissfliegenarten *Lucilia sericata* und *L. cuprina* werden in kleinen Portionen (30 bis 50 Eier) in Reagenzgläser gegeben, in denen zuvor 4 ml Nährmedium mit 1 ml Testlösung in der für die Endkonzentration notwendigen Zwischenverdünnung der Aktivsubstanz vermischt worden sind. Nach Beimpfung des Kulturmediums werden die Testgläser mit einem Wattestopfen verschlossen und im Brutschrank bei 30 °C über 4 Tage bebrütet. Im unbehandelten Medium entwickeln sich bis zu diesem Zeitpunkt ca. 1 cm lange Larven (Stadium 3). Ist eine Substanz aktiv, so sind die Larven zu diesem Zeitpunkt entweder tot oder deutlich zurückgeblieben. Der Versuch wird simultan, mit 4 Konzentrationen von 100, 40, 16 und 6,4 ppm durchgeführt. Die Wirksamkeit wird gemessen an der tiefsten noch voll wirksamen Konzentration (LC 100).

Die gute larvizide Wirkung der Verbindungen Nr. 1.1, 1.6, 1.8 und 1.9 der Formel I zeigt sich in diesem Test durch 100 %ige Mortalität der Larven nach spätestens 96 h bei Konzentrationen von 6,4 ppm.

B-7) Wirkung gegen *Musca domestica*

Je 50 g frisch zubereitetes CSMA-Nährsubstrat für Maden werden in einen Becher eingewogen. Von einer 1 gew.%igen acetonischen Lösung des Wirkstoffes werden 5 ml auf das im Becher befindliche Nährsubstrat pipettiert. Nach dem Durchmischen des Substrates lässt man das Aceton mindestens 20 Stunden lang verdampfen.

Dann werden 25 eintägige Maden von *Musca domestica* in den Becher mit dem so behandelten Nährsubstrat gegeben. Nachdem sich die Maden verpuppt haben, werden die gebildeten Puppen durch Ausschwemmen mit Wasser von dem Substrat abgetrennt. Die Anzahl der ausgeschwemmten Puppen spiegelt den toxischen Einfluss des Wirkstoffes auf die Madenentwicklung wider.

Die Puppen werden in einem mit einem Siebdeckel verschlossenen Gefäss deponiert. Nach 10 Tagen wird die Anzahl der aus den Puppen geschlüpften Fliegen bestimmt.

Die Verbindungen der Formel 1 zeigen gute Wirkung im obigen Test bei Konzentrationen über 40 ppm.

B-8a) Insektizide Frassgift-Wirkung

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 100 ppm der zu prüfenden Verbindung enthält.

Nach dem Antrocknen des Belages werden die Baumwollpflanzen mit *Spodoptera littoralis*-Larven im ersten larvalen Stadium besetzt. Der Versuch wird bei 26 °C und 50 % relativer Luftfeuchtigkeit durchgeführt. In Abständen von jeweils 24 Stunden werden Mortalität sowie Entwicklungs-und Häutungsstörungen der angesetzten Larven bestimmt.

Verbindungen gemäss Formel I, wie beispielsweise die Verbindungen 1.1, 1.3, 1.4, 1.6, 1.7, 1.8 und 1.9, zeigen mehr als 80 % Wirkung bei einer Konzentration von 100 ppm.

B-8b) Ovizide Wirkung auf *Spodoptera littoralis*

Auf Filterpapier abgelegte Eier von *Spodoptera littoralis* werden aus dem Papier ausgeschnitten und in eine 0,1 gew.%ige Lösung des Wirkstoffes in einem Aceton/Wasser-Gemisch (1:1) getaucht. Die so behandelten Eiablagen werden dann aus diesem Gemisch herausgenommen und bei 28 °C und 60 % relativer Luftfeuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen gemäss Formel I zeigen gute Wirkung im obigen Test.

B-9) Wirkung gegen Flöhe

Hunde mit der nachgewiesenen Fähigkeit, eine Flohpopulation permanent aufrechterhalten zu können, werden mit 100 Katzenflöhen, *Ctenocephalides felis* Bouche, infiziert. Die infizierten Hunde werden dann in 3 Gruppen zu je 2 Tieren eingeteilt. Die Hunde von 2 Gruppen werden an 10 aufeinanderfolgenden Tagen oral mit je 5 mg Wirksubstanz/kg Körpergewicht behandelt. Die Aktivsubstanz wird in Form einer wässrigen Suspension (mittels Spritzt) verabreicht. Die dritte Gruppe von Tieren bleibt unbehandelt und dient als Kontrollgruppe.

Nach 3,8 und 10 Tagen werden die Floheier von dem Papier, das sich unter den Hundeboxen befindet, aufgesammelt. Die Eier werden gezählt, auf ein für die Anzucht von Larven geeignetes Medium gegeben und inkubiert. Die Anzahl der gebildeten Puppen und ausgewachsenen Flöhe wird bestimmt.

Verbindungen gemäss Tabelle 1, wie beispielsweise Verbindungen 1.1, 1.6 und 1.12, zeigen mehr als 50 % Wirkung bei einer Dosis von 50 mg $kg^{-1}$ $Tag^{-1}$.

B-10) Vergleich mit dem Stand der Technik

Der Vergleich der vorliegenden Erfindung mit dem Stand der Technik erfolgt an Hand der beiden nachfolgend genannten Grundvertreter (A) und (B).

Verbindung des Standes der Technik:

(A) 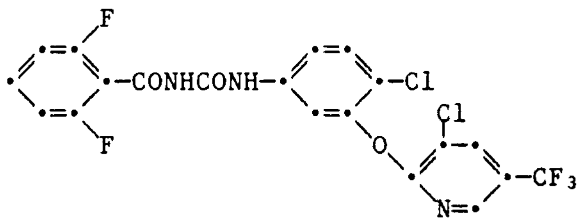 Verbindung Nr. 17 von EP 0 079 311

Verbindung der vorliegenden Erfindung:

(B) 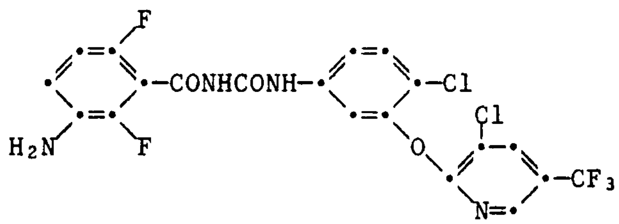 Verbindung Nr. 1.1

(a) Physikochemisches Verhalten der Substanzen (A) und (B)

| Löslichkeiten: | (A) | (B) |
|---|---|---|
| Wasser (ppm) | 0,02 | 0,11 |
| Hexan (ppm) | 3 | ~ 60 |
| n-Oktanol (%) | 0,065 | 0,56 |
| Isopropanol (%) | 0,09 | 0,30 |

(b) Bioverfügbarkeit der Substanzen (A) und (B)

Als Mass für die Bioverfügbarkeit wird der Blutplasmaspiegel der Aktivsubstanzen an mehreren Tagen nach der Injektion von je 1 mg Aktivsubstanz/kg Körpergewicht in Kälber bestimmt.

| Blutplasmaspiegel [ppb Aktivsubstanz] | | | | |
|---|---|---|---|---|
| Tag | (A) | | (B) | |
| | Kalb 1 | Kalb 2 | Kalb 3 | Kalb 4 |
| 1 | 7 | 5 | 126 | 185 |
| 7 | 3 | 4 | 48 | 66 |
| 14 | 4 | 9 | 16 | 27 |
| 21 | 7 | 10 | 10 | 12 |
| 28 | 6 | 5 | 5 | 6 |
| 35 | 6 | 10 | 2 | 4 |
| 42 | 9 | 6 | 0 | 0 |
| 49 | 4 | 4 | 0 | 0 |
| 56 | 5 | 5 | 0 | 0 |
| 63 | 4 | 6 | | 0 |
| 70 | 7 | 5 | | 0 |
| 77 | 5 | 2 | | 0 |
| 84 | 7 | 7 | | 0 |

1. Die Konzentration der Aktivsubstanz (A) (Stand der Technik) im Blut erreicht Höchstwerte zwischen 7 und 10 ppb. Dieser Blutplasmaspiegel wird zwar über einen längeren Zeitraum aufrecht erhalten, sein absoluter Wert ist jedoch als niedrig anzusehen.

2. Bei Verabreichung der Aktivsubstanz (B) (Verbindung 1.1 der vorliegenden Erfindung) wird sehr rasch eine hohe Konzentration im Blutplasma erreicht. Darüberhinaus sind nach 35 bis 42 Tagen bereits keine messbaren Aktivsubstanzanteile mehr nachweisbar.

Somit führt Substanz (B) sehr rasch nach Applikation zu höheren Blutplasmawerten und zu einer deutlich besseren Bioverfügbarkeit als Substanz (A). (B) wird darüberhinaus auch wesentlich rascher ausgeschieden als (A) und führt damit zu einer geringeren Belastung des behandelten Nutztieres.

## Patentansprüche

**1.** Verbindungen der Formel I

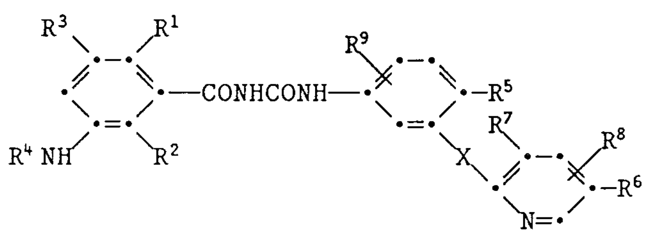

(I)

worin

$R^1$, $R^2$, $R^3$, $R^7$, $R^8$ und $R^9$ unabhängig voneinander H oder Halogen,

$R^4$ H, $R^{10}CO$- oder $R^{11}NHCO$-, wobei $R^{10}$ für ein $C_1$-$C_4$-Alkyl steht, welches unsubstituiert oder mit einem bis drei gleichen oder verschiedenen Substituenten aus der Gruppe Halogen, $C_1$-$C_4$-Alkoxy, $C_1$-$C_4$-Acyloxy und -COOG substituiert ist, worin G H, ein Alkali-oder Erdalkalimetallkation bedeutet, und $R^{11}$ für eine unsubstituierte oder durch Halogen substituierte $C_1$-$C_4$-Alkyl- oder Phenylgruppe steht,

$R^5$ und $R^6$ unabhängig voneinander H, Halogen, $C_1$-$C_6$-Alkyl oder $C_1$-$C_6$-Haloalkyl

und

X O, $S(O)_n$ oder NH, wobei n für 0, 1 oder 2 steht,

bedeuten.

**2.** Verbindungen gemäss Anspruch 1, worin $R^4$ Wasserstoff und $R^5$ und $R^6$ unabhängig voneinander Wasserstoff, Halogen, $C_1$-$C_4$-Alkyl oder $C_1$-$C_4$-Haloalkyl bedeuten und $R^1$, $R^2$, $R^3$, $R^7$ , $R^8$ , $R^9$ und X

die im Anspruch 1 angegebenen Bedeutungen haben.

**3.** Verbindungen gemäss Anspruch 1, worin $R^4$ $R^{10}CO$- oder $R^{11}NHCO$-bedeutet und $R^1$, $R^2$, $R^3$, $R^5$ bis $R^{11}$ sowie X die im Anspruch 1 angegebenen Bedeutungen haben.

**4.** Verbindungen gemäss Anspruch 1, worin $R^4$ Wasserstoff und X Sauerstoff bedeuten und $R^1$, $R^2$, $R^3$ und $R^5$ bis $R^{11}$ die in Anspruch 1 angegebenen Bedeutungen haben.

**5.** Verbindungen gemäss Anspruch 1, worin $R^4$ Wasserstoff und X $S(O)_n$ bedeuten und $R^1$, $R^2$, $R^3$, $R^5$ bis $R^{11}$ sowie n die in Anspruch 1 angegebenen Bedeutungen haben.

**6.** Verbindungen gemäss Anspruch 1, worin $R^4$ Wasserstoff und X NH bedeuten und $R^1$ , $R^2$, $R^3$ , $R^5$ bis $R^{11}$ die in Anspruch 1 angegebenen Bedeutungen haben.

**7.** Verbindungen gemäss Anspruch 1, worin $R^1$ und $R^2$ unabhängig voneinander Fluor oder Chlor, $R^3$ Wasserstoff, Fluor oder Chlor, $R^4$ Wasserstoff, $R^{10}CO$- oder $R^{11}NHCO$-, wobei $R^{10}$ für unsubstituiertes oder durch -COOG substituiertes $C_1$-$C_4$-Alkyl steht, $R^5$ und $R^6$ unabhängig voneinander Wasserstoff, Halogen, $C_1$-$C_2$-Alkyl oder $C_1$-$C_2$-Haloalkyl und $R^7$, $R^8$ sowie $R^9$ unabhängig voneinander Wasserstoff, Fluor, Brom oder Chlor bedeuten und $R^{11}$, G sowie X die im Anspruch 1 angegebenen Bedeutungen haben.

**8.** Verbindungen gemäss Anspruch 7, worin $R^1$ und $R^2$ Fluor bedeuten.

**9.** Verbindungen gemäss Anspruch 7, worin $R^3$ und $R^4$ Wasserstoff bedeuten.

**10.** Verbindungen gemäss Anspruch 1, worin $R^1$ und $R^2$ Fluor bedeuten und $R^3$ bis $R^9$ sowie X die in Anspruch 1 angegebenen Bedeutungen haben.

**11.** Verbindungen gemäss Anspruch 1, worin $R^1$ und $R^2$ Fluor, $R^3$ und $R^4$ Wasserstoff, $R^5$ Wasserstoff, Methyl, $CF_3$, Fluor, Chlor oder Brom, $R^6$ Wasserstoff oder $C_1$-$C_2$-Haloalkyl und $R^7$, $R^8$ sowie $R^9$ unabhängig voneinander Wasserstoff, Fluor oder Chlor bedeuten und X die in Anspruch 1 angegebene Bedeutung hat.

**12.** Verbindungen gemäss Anspruch 11, worin X Sauerstoff bedeutet.

**13.** Verbindungen gemäss Anspruch 11, worin X Schwefel bedeutet.

**14.** Verbindungen gemäss Anspruch 11, worin X NH bedeutet.

**15.** Verbindungen gemäss Anspruch 1, worin $R^1$ und $R^2$ Fluor, $R^3$ und $R^4$ Wasserstoff, $R^5$ Fluor oder Chlor, $R^6$ $CF_3$, $R^7$ Chlor, $R^8$ und $R^9$ Wasserstoff und X Sauerstoff bedeuten.

**16.** Verbindungen gemäss Anspruch 1, worin $R^1$ und $R^2$ Fluor, $R^3$ und $R^4$ Wasserstoff, $R^5$ Fluor oder Chlor, $R^6$ $CF_3$, $R^7$ Chlor, $R^8$ und $R^9$ Wasserstoff und X Schwefel bedeuten.

**17.** Verbindungen gemäss Anspruch 1, worin $R^1$ und $R^2$ Fluor, $R^3$ und $R^4$ Wasserstoff, $R^5$ Fluor oder Chlor, $R^6$ $CF_3$, $R^7$ Chlor, $R^8$ und $R^9$ Wasserstoff und X NH bedeuten.

**18.** Verbindungen gemäss Anspruch 16, worin $R^3$ Halogen, $R^7$ Fluor sowie $R^8$ und $R^9$ unabhängig voneinander Fluor oder Chlor bedeuten und $R^1$, $R^2$ sowie $R^4$ , $R^5$, $R^6$ und X die in Anspruch 16 angegebene Bedeutung haben.

**19.** N-[3-(3-Chlor-5-trifluormethyl-pyridyl-2-oxy)-4-fluorphenyl]-N'-[2,6-difluor-3-aminobenzoyl]-harnstoff,
N-[3-(3-Chlor-5-trifluormethyl-pyridyl-2-oxy)-4-chlorphenyl]-N'-[2,6-dichlor-3-aminobenzoyl]-harnstoff,
N-[3-(3-Chlor-5-trifluormethyl-pyridyl-2-oxy)-4-chlorphenyl]-N'-[2,6-difluor-3-aminobenzoyl]-harnstoff,
N-[3-(3-Chlor-5-trifluormethyl-pyridyl-2-oxy)-4-chlorphenyl]-N'-[2,6-difluor-3-(N-methylcarbamoylamino)-benzoyl]-harnstoff,
N-[3-(3-Chlor-5-trifluormethyl-pyridyl-2-oxy)-4-chlorphenyl]-N'-[2,6-difluor-3-(N-acetylcarbamoylamino)-benzoyl]-harnstoff,
N-[3-(3-Chlor-5-trifluormethyl-pyridyl-2-oxy)-4-chlorphenyl)-N'-[2,6-difluor-3-(N-carboxypropionylamino)-benzoyl]-harnstoff,
N-[3-(3-Chlor-5-trifluormethyl-pyridyl-2-amino)-4-chlorphenyl]-N'-[2,6-difluor-3-aminobenzoyl]-harnstoff und
N-[3-(3-Chlor-5-trifluormethyl-pyridyl-2-thio)-4-chlorphenyl]-N'-[2,6-difluor-3-aminobenzoyl]-harnstoff nach Anspruch 1.

**20.** N-[3-(3-Chlor-5-trifluormethyl-pyridyl-2-oxy)-4-chlorphenyl]-N'-[2,6-difluor-3-aminobenzoyl]-harnstoff nach Anspruch 1.

**21.** N-[3-(3-Chlor-5-trifluormethyl-pyridyl-2-oxy)-4-fluorphenyl]-N'-[2,6-difluor-3-aminobenzoyl]-harnstoff nach Anspruch 1.

**22.** N-[3-(3-Chlor-5-trifluormethyl-pyridyl-2-thio)-4-chlorphenyl]-N'-[2,6-difluor-3-aminobenzoyl]-harnstoff nach Anspruch 1.

**23.** Verbindungen der Formel II

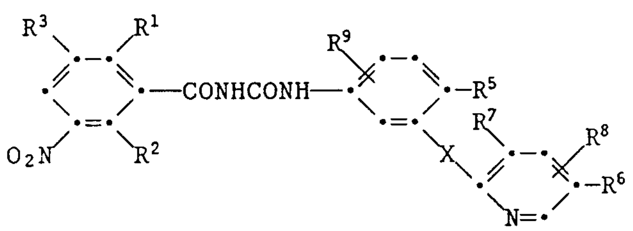

(II)

worin
$R^1$, $R^2$, $R^3$, $R^7$, $R^8$ und $R^9$ unabhängig voneinander H oder Halogen,
$R^5$ und $R^6$ unabhängig voneinander H, Halogen, $C_1$-$C_6$-Alkyl oder $C_1$-$C_6$-Haloalkyl
und
X C, $S(O)_n$ oder NH, wobei n für 0, 1 oder 2 steht,
bedeuten.

**24.** N-[3-(3-Chlor-5-trifluormethyl-pyridyl-2-oxy)-4-chlorphenyl]-N'-[2,6-difluor-3-nitrobenzoyl]-harnstoff,
N-[3-(3-Chlor-5-trifluormethyl-pyridyl-2-oxy)-4-fluorphenyl]-N'-[2,6-difluor-3-nitrobenzoyl]-harnstoff und
N-[3-(3-Chlor-5-trifluormethyl-pyridyl-2-oxy)-4-chlorphenyl]-N'-[2,6-dichlor-3-nitrobenzoyl]-harnstoff nach Anspruch 23.

**25.** Verbindungen der Formel IV

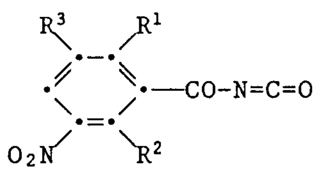

(IV)

worin
$R^1$, $R^2$ und $R^3$ unabhängig voneinander Wasserstoff oder Halogen bedeuten;
jedoch nicht gleichzeitig für Wasserstoff stehen.

**26.** Verbindungen der Formel VI

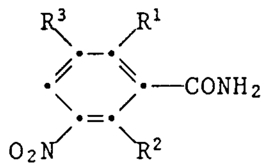

(VI)

worin
$R^1$, $R^2$ und $R^3$ unabhängig voneinander Wasserstoff oder Halogen bedeuten;
mit Ausnahme von Verbindungen, worin $R^3$ Wasserstoff bedeutet und $R^1$ und $R^2$ gleichzeitig für Chlor stehen.

**27.** 2,6-Difluor-3-nitro-benzoesäureamid nach Anspruch 26.

**28.** Verfahren zur Herstellung von Verbindungen der Formel I

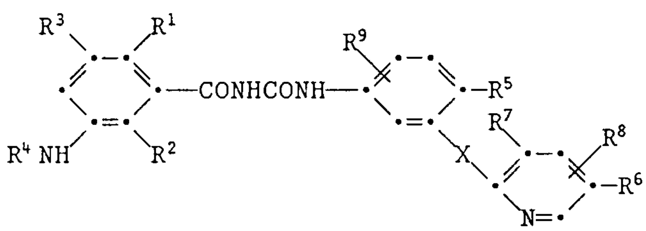

(I)

worin

| | |
|---|---|
| $R^1$, $R^2$, $R^3$, $R^7$, $R^8$ und $R^9$ | unabhängig voneinander H oder Halogen, |
| $R^4$ | H, $R^{10}$CO- oder $R^{11}$NHCO-, wobei $R^{10}$ für ein $C_1$-$C_4$-Alkyl steht, welches unsubstituiert oder mit einem bis drei gleichen oder verschiedenen Substituenten aus der Gruppe Halogen, $C_1$-$C_4$-Alkoxy, $C_1$-$C_4$-Acyloxy und -COOG substituiert ist, worin G H, ein Alkali-oder Erdalkalimetallkation bedeutet, und $R^{11}$ für eine unsubstituierte oder durch Halogen substituierte $C_1$-$C_4$-Alkyl- oder Phenylgruppe steht, |
| $R^5$ und $R^6$ | unabhängig voneinander H, Halogen, $C_1$-$C_6$-Alkyl oder $C_1$-$C_6$-Haloalkyl |

und
X O, $S(O)_n$ oder NH, wobei n für 0, 1 oder 2 steht, bedeuten, dadurch gekennzeichnet, dass man

a) zur Herstellung einer Verbindung der Formel I, in welcher $R^4$ für Wasserstoff steht, eine Verbindung der Formel II

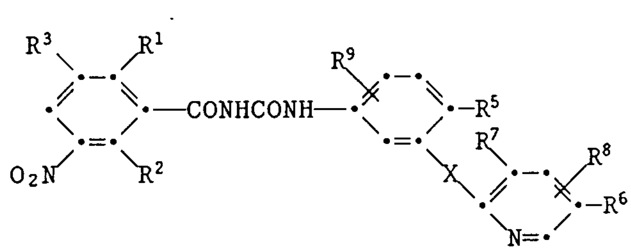

(II)

worin $R^1$ bis $R^9$ und X die für Formel I angegebenen Bedeutungen haben, i) in Anwesenheit eines geeigneten Katalysators und bei Normaldruck Wasserstoff hydriert oder ii) chemisch reduziert oder

b) zur Herstellung von Verbindungen der Formel I, in denen
$R^4$ $R^{10}$CO- oder $R^{11}$NHCO-, wobei $R^{10}$ für ein $C_1$-$C_4$-Alkyl steht, welches unsubstituiert oder

mit einem bis drei gleichen oder verschiedenen Substituenten aus der Gruppe Halogen, $C_1$-$C_4$-Alkoxy, $C_1$-$C_4$-Acyloxy und -COOG substituiert ist, worin G H, ein Alkali- oder Erdalkalimetallkation bedeutet, und $R^{11}$ für eine unsubstituierte oder durch Halogen substituierte $C_1$-$C_4$-Alkyl- oder Phenylgruppe steht, bedeutet,

eine Verbindung der Formel I, worin $R^4$ Wasserstoff bedeutet und $R^1$, $R^2$, $R^3$, $R^5$ bis $R^9$ und X die oben angegebenen Bedeutungen haben, mit einer Säure $R^{10}COOH$, einem ihrer Säurehalogenide, ihrem Säureanhydrid oder mit einem Isocyanat $R^{11}NCO$ umsetzt, wobei $R^{10}$ und $R^{11}$ wie unter b) definiert sind und die Verbindungen der Formel II dadurch erhältlich sind, dass man entweder

(a) ein Anilin der Formel III

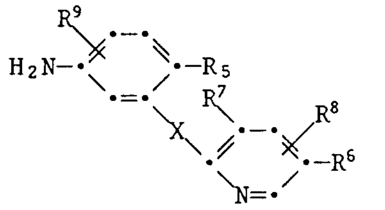

(III)

worin $R^5$ bis $R^9$ und X die obenstehenden Bedeutungen haben,
mit einem Benzoylisocyanat der Formel IV umsetzt

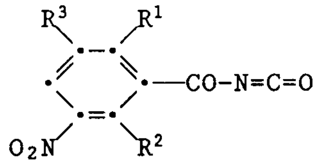

(IV)

worin $R^1$, $R^2$ und $R^3$ die obenstehenden Bedeutungen haben oder
(b) ein Isocyanat der Formel V

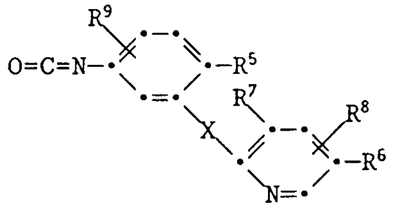

(V)

worin $R^5$ bis $R^9$ und X die obenstehenden Bedeutungen haben,
mit einem Benzamid der Formel VI umsetzt

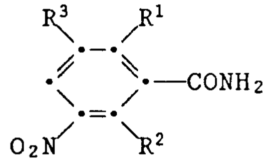

(VI)

worin $R^1$, $R^2$ und $R^3$ die obenstehenden Bedeutungen haben,
oder

(c) ein Anilin der Formel III mit einem Urethan der Formel VII umsetzt

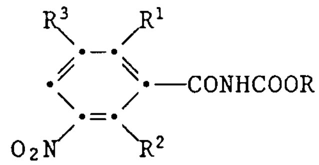

(VII)

worin $R^1$, $R^2$ und $R^3$ die obenstehenden Bedeutungen haben und R $C_1$-$C_5$-Alkyl oder unsubstituiertes oder durch Nitro substituiertes Phenyl bedeutet.

**29.** Mittel zur Bekämpfung von Ekto- und Endoparasiten am Haus- und Nutztier oder zur Bekämpfung von Schadinsekten, dadurch gekennzeichnet, dass es neben den üblichen Formulierungshilfsstoffen als Wirkstoff eine Verbindung der Formel I gemäss Anspruch 1 enthält.

**30.** Verfahren zur Bekämpfung von Nutz- und Haustiere befallenden Parasiten oder von Schadinsekten, dadurch gekennzeichnet, dass man eine parasitizid bzw. insektizid wirksame Menge mindestens einer Verbindung der Formel I gemäss Anspruch 1 auf den Parasiten, das Schadinsekt oder deren Lebensraum appliziert.

**31.** Verbindungen der Formel I gemäss einem der Ansprüche 1 bis 22 zur Bekämpfung von Parasiten am Nutz- oder Haustier.

## Claims

**1.** A compound of formula I

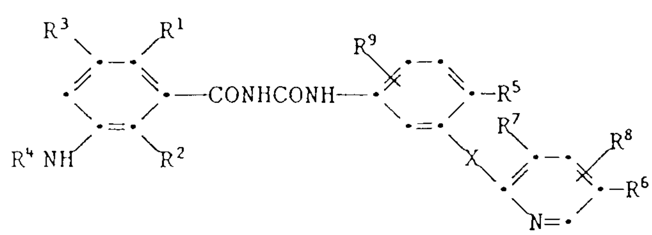

(I)

wherein
each of $R^1$, $R^2$, $R^3$, $R^7$, $R^8$ and $R^9$, independently of the others, is H or halogen,
$R^4$ is H, $R^{10}$CO- or $R^{11}$NHCO- wherein $R^{10}$ is a $C_1$-$C_4$alkyl group that is unsubstituted or substituted by from one to three identical or different substituents selected from the group consisting of halogen, $C_1$-$C_4$alkoxy, $C_1$-$C_4$acyloxy and -COOG, wherein G is H, an alkali metal cation or an alkaline earth metal cation, and $R^{11}$ is an unsubstituted or halo-substituted $C_1$-$C_4$alkyl or phenyl group,
each of $R^5$ and $R^6$, independently of the other, is H, halogen, $C_1$-$C_6$alkyl or $C_1$-$C_6$haloalkyl and
X is O, $S(O)_n$ or NH wherein n is 0, 1 or 2.

**2.** A compound according to claim 1, wherein $R^4$ is hydrogen and each of $R^5$ and $R^6$, independently of the other, is hydrogen, halogen, $C_1$-$C_4$alkyl or $C_1$-$C_4$haloalkyl and $R^1$, $R^2$, $R^3$, $R^7$, $R^8$, $R^9$ and X are as defined in claim 1.

**3.** A compound according to claim 1, wherein $R^4$ is $R^{10}$CO-or $R^{11}$NHCO- and $R^1$, $R^2$, $R^3$, $R^5$ to $R^{11}$ and X are as defined in claim 1.

**4.** A compound according to claim 1, wherein $R^4$ is hydrogen and X is oxygen and $R^1$, $R^2$, $R^3$ and $R^5$ to $R^{11}$ are as defined in claim 1.

**5.** A compound according to claim 1, wherein $R^4$ is hydrogen and X is $S(O)_n$ and $R^1$, $R^2$, $R^3$, $R^5$ to $R^{11}$ and n are as defined in claim 1.

**6.** A compound according to claim 1, wherein $R^4$ is hydrogen and X is NH and $R^1$, $R^2$, $R^3$ and $R^5$ to $R^{11}$ are as defined in claim 1.

**7.** A compound according to claim 1, wherein each of $R^1$ and $R^2$, independently of the other, is fluorine or chlorine, $R^3$ is hydrogen, fluorine or chlorine, $R^4$ is hydrogen, $R^{10}$CO- or $R^{11}$NHCO- wherein $R^{10}$ is $C_1$-$C_4$alkyl that is unsubstituted or substituted by -COOG, each of $R^5$ and $R^6$, independently of the other, is hydrogen, halogen, $C_1$-$C_2$alkyl or $C_1$-$C_2$haloalkyl and each of $R^7$, $R^8$ and $R^9$, independently of the others, is hydrogen, fluorine, bromine or chlorine and $R^{11}$, G and X are as defined in claim 1.

**8.** A compound according to claim 7, wherein $R^1$ and $R^2$ are fluorine.

**9.** A compound according to claim 7, wherein $R^3$ and $R^4$ are hydrogen.

**10.** A compound according to claim 1, wherein $R^1$ and $R^2$ are fluorine and $R^3$ to $R^9$ and X are as defined in claim 1.

**11.** A compound according to claim 1, wherein $R^1$ and $R^2$ are fluorine, $R^3$ and $R^4$ are hydrogen, $R^5$ is hydrogen, methyl, $CF_3$, fluorine, chlorine or bromine, $R^6$ is hydrogen or $C_1$-$C_2$haloalkyl and each of $R^7$, $R^8$ and $R^9$, independently of the others, is hydrogen, fluorine or chlorine and X is as defined in claim 1.

**12.** A compound according to claim 11, wherein X is oxygen.

**13.** A compound according to claim 11, wherein X is sulfur.

**14.** A compound according to claim 11, wherein X is NH.

**15.** A compound according to claim 1, wherein $R^1$ and $R^2$ are fluorine, $R^3$ and $R^4$ are hydrogen, $R^5$ is fluorine or chlorine, $R^6$ is $CF_3$, $R^7$ is chlorine, $R^8$ and $R^9$ are hydrogen and X is oxygen.

**16.** A compound according to claim 1, wherein $R^1$ and $R^2$ are fluorine, $R^3$ and $R^4$ are hydrogen, $R^5$ is fluorine or chlorine, $R^6$ is $CF_3$, $R^7$ is chlorine, $R^8$ and $R^9$ are hydrogen and X is sulfur.

**17.** A compound according to claim 1, wherein $R^1$ and $R^2$ are fluorine, $R^3$ and $R^4$ are hydrogen, $R^5$ is fluorine or chlorine, $R^6$ is $CF_3$, $R^7$ is chlorine, $R^8$ and $R^9$ are hydrogen and X is NH.

**18.** A compound according to claim 16, wherein $R^3$ is halogen, $R^7$ is fluorine and each of $R^8$ and $R^9$, independently of the other, is fluorine or chlorine and $R^1$, $R^2$ and $R^4$, $R^5$, $R^6$ and X are as defined in claim 16.

**19.** N-[3-(3-chloro-5-trifluoromethylpyridyl-2-oxy)-4-fluorophenyl]-N'-[2,6-difluoro-3-aminobenzoyl]-urea,
N-[3-(3-chloro-5-trifluoromethylpyridyl-2-oxy)-4-chlorophenyl]-N'-[2,6-dichloro-3-aminobenzoyl]-urea,
N-[3-(3-chloro-5-trifluoromethylpyridyl-2-oxy)-4-chlorophenyl]-N'-[2,6-difluoro-3-aminobenzoyl]-urea,
N-[3-(3-chloro-5-trifluoromethylpyridyl-2-oxy)-4-chlorophenyl]-N'-[2,6-difluoro-3-(N-methylcarbamoylamino)-benzoyl]-urea,
N-[3-(3-chloro-5-trifluoromethylpyridyl-2-oxy)-4-chlorophenyl]-N'-[2,6-difluoro-3-(N-acetylcarbamoylamino)-benzoyl]-urea,
N-[3-(3-chloro-5-trifluoromethylpyridyl-2-oxy)-4-chlorophenyl]-N'-[2,6-difluoro-3-(N-carboxypropionylamino)-benzoyl]-urea,
N-[3-(3-chloro-5-trifluoromethylpyridyl-2-amino)-4-chlorophenyl]-N'-[2,6-difluoro-3-aminobenzoyl]-urea and
N-[3-(3-chloro-5-trifluoromethylpyridyl-2-thio)-4-chlorophenyl]-N'-[2,6-difluoro-3-aminobenzoyl]-urea according to claim 1.

**20.** N-[3-(3-chloro-5-trifluoromethylpyridyl-2-oxy)-4-chlorophenyl]-N'-[2,6-difluoro-3-aminobenzoyl]-urea according to claim 1.

**21.** N-[3-(3-chloro-5-trifluoromethylpyridyl-2-oxy)-4-fluorophenyl]-N'-[2,6-difluoro-3-aminobenzoyl]-urea according to claim 1.

**22.** N-[3-(3-chloro-5-trifluoromethylpyridyl-2-thio)-4-chlorophenyl]-N'-[2,6-difluoro-3-aminobenzoyl]-urea according to claim 1.

**23.** A compound of formula II

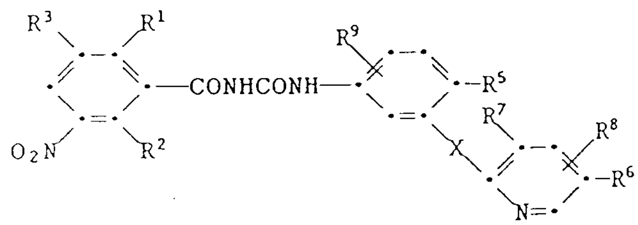

(II)

wherein
each of $R^1$, $R^2$, $R^3$, $R^7$, $R^8$ and $R^9$, independently of the others, is H or halogen,
each of $R^5$ and $R^6$, independently of the other, is H, halogen, $C_1$-$C_6$alkyl or $C_1$-$C_6$haloalkyl and
X is O, $S(O)_n$ or NH wherein n is 0, 1 or 2.

**24.** N-[3-(3-chloro-5-trifluoromethylpyridyl-2-oxy)-4-chlorophenyl]-N'-[2,6-difluoro-3-nitrobenzoyl]-urea,
N-[3-(3-chloro-5-trifluoromethylpyridyl-2-oxy)-4-fluorophenyl]-N'-[2,6-difluoro-3-nitrobenzoyl]-urea and
N-[3-(3-chloro-5-trifluoromethylpyridyl-2-oxy)-4-chlorophenyl]-N'-[2,6-dichloro-3-nitrobenzoyl]-urea according to claim 23.

**25.** A compound of formula IV

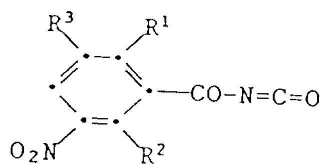

(IV)

wherein
$R^1$, $R^2$ and $R^3$ are each independently of the others hydrogen or halogen; but are not simultaneously hydrogen.

**26.** A compound of formula VI

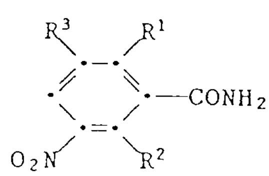

(VI)

wherein
each of $R^1$, $R^2$ and $R^3$, independently of the others, is hydrogen or halogen; with the exception of a compound wherein $R^3$ is hydrogen and $R^1$ and $R^2$ are simultaneously chlorine.

**27.** 2,6-difluoro-3-nitrobenzoic acid amide according to claim 26.

**28.** A process for the preparation of a compound of formula I

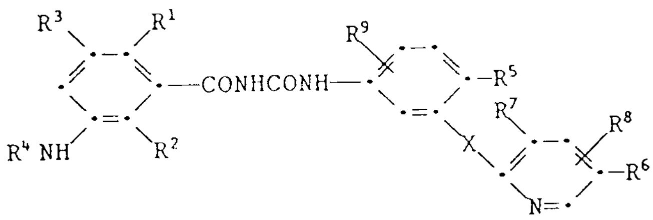

(I)

wherein
each of $R^1$, $R^2$, $R^3$, $R^7$, $R^8$ and $R^9$, independently of the others, is H or halogen,
$R^4$ is H, $R^{10}CO$- or $R^{11}NHCO$- wherein $R^{10}$ is a $C_1$-$C_4$alkyl group that is unsubstituted or substituted by from one to three identical or different substituents selected from the group consisting of halogen, $C_1$-$C_4$alkoxy, $C_1$-$C_4$acyloxy and -COOG, wherein G is H, an alkali metal cation or an alkaline earth metal cation, and $R^{11}$ is an unsubstituted or halo-substituted $C_1$-$C_4$alkyl or phenyl group,
each of $R^5$ and $R^6$, independently of the other, is H, halogen, $C_1$-$C_6$alkyl or $C_1$-$C_6$haloalkyl and
X is O, $S(O)_n$ or NH wherein n is 0, 1 or 2,
which comprises

a) for the preparation of a compound of formula I wherein $R^4$ is hydrogen, i) hydrogenating a compound of formula II

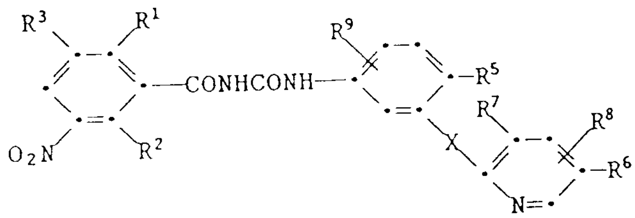

(II),

wherein $R^1$ to $R^9$ and X are as defined for formula I, in the presence of a suitable catalyst and at normal pressure of hydrogen, or ii) subjecting it to chemical reduction, or

b) for the preparation of a compound of formula I wherein $R^4$ is $R^{10}CO$- or $R^{11}NHCO$- wherein $R^{10}$ is a $C_1$-$C_4$alkyl group that is unsubstituted or substituted by from one to three identical or different substituents selected from the group consisting of halogen, $C_1$-$C_4$alkoxy, $C_1$-$C_4$acyloxy and -COOG, wherein G is H, an alkali metal cation or an alkaline earth metal cation, and $R^{11}$ is an unsubstituted or halo-substituted $C_1$-$C_4$alkyl or phenyl group,

reacting a compound of formula I wherein $R^4$ is hydrogen and $R^1$, $R^2$, $R^3$, $R^5$ to $R^9$ and X are as defined above, with an acid $R^{10}COOH$, an acid halide thereof, an acid anhydride thereof or with an isocyanate $R^{11}NCO$, $R^{10}$ and $R^{11}$ being as defined under b) and the compounds of formula II being obtainable by reacting either

(a) an aniline of formula III

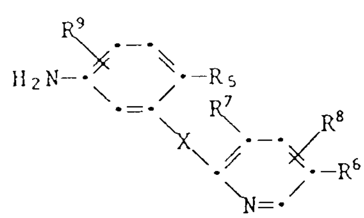

(III)

wherein $R^5$ to $R^9$ and X are as defined above, with a benzoyl isocyanate of formula IV

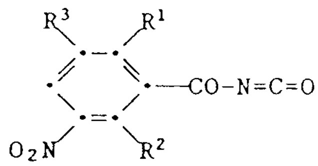

(IV)

wherein $R^1$, $R^2$ and $R^3$ are as defined above, or
(b) an isocyanate of formula V

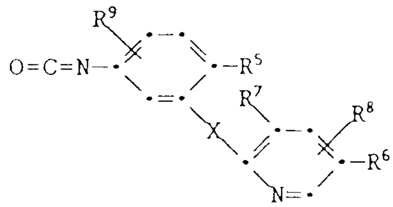

(V)

wherein $R^5$ to $R^9$ and X are as defined above, with a benzamide of formula VI

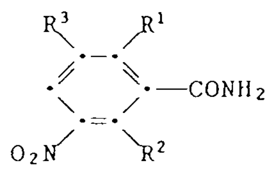

(VI)

wherein $R^1$, $R^2$ and $R^3$ are as defined above, or
(c) an aniline of formula III with a urethane of formula VII

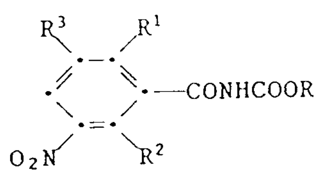

(VII)

wherein $R^1$, $R^2$ and $R^3$ are as defined above and R is $C_1$-$C_5$alkyl or unsubstituted or nitro-substituted phenyl.

**29.** A composition for controlling ectoparasites and endoparasites in and on domestic animals and productive livestock or for controlling noxious insects, which comprises as active ingredient a compound of formula I according to claim 1 together with customary formulation adjuvants.

**30.** A method of controlling parasites that attack productive livestock and domestic animals or of controlling noxious insects, which comprises applying a parasiticidally or insecticidally effective amount of at least one compound of formula I according to claim 1 to the parasite, the noxious insect or the locus thereof.

**31.** A compound of formula I according to any one of claims 1 to 22 for controlling parasites in and on productive livestock or domestic animals.

## Revendications

**1.** Composés de formule I

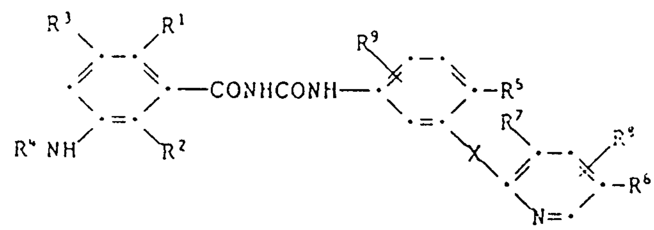

dans laquelle

$R^1$ , $R^2$ , $R^3$ , $R^7$ , $R^8$ et $R^9$ représentent indépendamment l'un de l'autre H ou un halogène,

$R^4$ représente H, $R^{10}$ représente CO-ou $R^{11}$ représente NHCO- , où $R^{10}$ représente un alkyle en $C_1$ à $C_4$ qui est non substitué ou substitué par de un à trois substituants identiques ou différents du groupe halogène, alcoxy en $C_1$ à $C_4$ , acyloxy en $C_1$ à $C_4$ et - COOG, où G représente H, un cation de métal alcalin ou alcalino-terreux, et $R^{11}$ représente un groupe alkyle en $C_1$ à $C_4$ ou phényle non substitué ou substitué par un halogène,

$R^5$ et $R^6$ représentent indépendamment l'un de l'autre H, un halogène, un alkyle en $C_1$ à $C_6$ ou un haloalkyle en $C_1$ à $C_6$ , et

X représente O, $S(O)_n$ ou NH, où n vaut 0, 1 ou 2.

**2.** Composés selon la revendication 1, où $R^4$ représente un hydrogène et $R^5$ et $R^6$ représentent indépendamment l'un' de l'autre un hydrogène, un halogène, un alkyle en $C_1$ à $C_4$ ou un haloalkyle en $C_1$ à $C_4$ et $R^1$ , $R^2$ , $R^3$ , $R^7$ , $R^8$ , $R^9$ et X ont les significations données dans la revendication 1.

**3.** Composés selon la revendication 1, où $R^4$ représente $R^{10}$CO- ou $R^{11}$NHCO- et $R^1$ , $R^2$ , $R^3$ , $R^5$ à $R^{11}$ ainsi que X ont les significations indiquées dans la revendication 1.

**4.** Composés selon la revendication 1, où $R^4$ représente un hydrogène et X un oxygène et $R^1$ , $R^2$ , $R^3$ et $R^5$ à $R^{11}$ ont les significations données dans la revendication 1.

**5.** Composés selon la revendication 1, où $R^4$ représente un hydrogène et X $S(O)_n$ et $R^1$ , $R^2$ , $R^3$ , $R^5$ à $R^{11}$ ainsi que n ont les significations indiquées dans la revendication 1.

**6.** Composés selon la revendication 1, où $R^4$ représente un hydrogène et X représente NH et $R^1$ , $R^2$ , $R^3$ , $R^5$ à $R^{11}$ ont les significations indiquées dans la revendication 1.

**7.** Composés selon la revendication 1, où $R^1$ et $R^2$ représentent indépendamment l'un de l'autre un fluor ou un chlore, $R^3$ un hydrogène, un fluor ou un chlore, $R^4$ un hydrogène, $R^{10}$ CO- ou $R^{11}$ NHCO- , où $R^{10}$ représente un alkyle en $C_1$ à $C_4$ non substitué ou substitué par -COOG, $R^5$ et $R^6$ représentent indépendamment l'un de l'autre un hydrogène, halogène, alkyle en $C_1$ à $C_2$ ou un haloalkyle en $C_1$ à $C_2$ et $R^7$ , $R^8$ ainsi que $R^9$ représentent indépendamment l'un de l'autre un hydrogène, fluor, brome ou chlore et $r^{11}$ , G ainsi que X ont les significations indiquées dans la revendication 1.

**8.** Composés selon la revendication 7, où $R^1$ et $R^2$ représentent un fluor.

**9.** Composés selon la revendication 7, où $R^3$ et $R^4$ représentent un hydrogène.

**10.** Composés selon la revendication 1, où $R^1$ et $R^2$ représentent un fluor et $R^3$ à $R^9$ ainsi que X ont les significations indiquées dans la revendication 1.

**11.** Composés selon la revendication 1, où $R^1$ et $R^2$ représentent un fluor, $R^3$ et $R^4$ représentent un hydrogène, $R^5$ un hydrogène, un méthyle, $CF_3$ , un fluor, un chlore ou un brome, $R^6$ représente un

hydrogène ou un haloalkyle en $C_1$ à $C_2$ et $R^7$ , $R^8$ ainsi que $R^9$ représentent indépendamment l'un de l'autre un hydrogène, un fluor ou un chlore et X a la signification donnée dans la revendication 1.

**12.** Composés selon la revendication 11, où X représente un oxygène.

**13.** Composés selon la revendication 11, où X représente un soufre.

**14.** Composés selon la revendication 11, où X représente NH.

**15.** Composés selon la revendication 1, où $R^1$ et $R^2$ représentent un fluor, $R^3$ et $R^4$ représentent un hydrogène, $R^5$ représente un fluor ou un chlore, $R^6$ représente $CF_3$ , $R^7$ un chlore, $R^8$ et $R^9$ un hydrogène et X un oxygène.

**16.** Composés selon la revendication 1, ou $R^1$ et $R^2$ représentent un fluor, $R^3$ et $R^4$ un hydrogène, $R^5$ un fluor ou un chlore, $R^6$ $CF_3$ , $R^7$ un chlore, $R^8$ et $R^9$ un hydrogène et X un soufre.

**17.** Composés selon la revendication 1, où $R^1$ et $R^2$ représentent un fluor, $R^3$ et $R^4$ un hydrogène, $R^5$ un fluor ou un chlore, $R^6$ représente $CF_3$ , $R^7$ un chlore, $R^8$ et $R^9$ un hydrogène et X représente NH.

**18.** Composés selon la revendication 16, où $R^3$ représente un halogène, $R^7$ un fluor, ainsi que $R^8$ et $R^9$, indépendamment l'un de l'autre, un fluor ou un chlore, et $R^1$ , $R^2$ ainsi que $R^4$ , $R^5$ , $R^6$ et X ont la signification donnée dans la revendication 16.

**19.** N-[3-(3-chloro-5-trifluorométhyl-pyridyl-2-oxy)-4-fluorophényl]-N'-[2,6-difluoro-3-aminobenzoyl]-urée,
N-[3-(3-chloro-5-trifluorométhyl-pyridyl-2-oxy)-4-chlorophényl]-N'-[2,6-dichloro-3-aminobenzoyl]-urée,
N-[3-(3-chloro-5-trifluorométhyl-pyridyl-2-oxy)-4-chlorophényl]-N'-[2,6-difluoro-3-aminobenzoyl]-urée,
N-[3-(3-chloro-5-trifluorométhyl-pyridyl-2-oxy)-4-chlorophényl]-N'-[2,6-difluoro-3-(N-méthylcarbamoylamino)-benzoyl]-urée,
N-[3-(3-chloro-5-trifluorométhyl-pyridyl-2-oxy)-4-chlorophényl]-N'-[2,6-difluoro-3-(N-acétylcarbamoylamino)-benzoyl]-urée,
N-[3-(3-chloro-5-trifluorométhyl-pyridyl-2-oxy)-4-chlorophényl]-N'-[2,6-difluoro-3-(N-carboxypropionylamino)-benzoyl]-urée,
N-[3-(3-chloro-5-trifluorométhyl-pyridyl-2-amino)-4-chlorophényl]-N'-[2,6-difluoro-3-aminobenzoyl]-urée et
N-[3-(3-chloro-(trifluorométhyl-pyridyl-2-thio)-4-chlorophényl]-N'-[2,6-difluoro-3-aminobenzoyl]-urée selon la revendication 1.

**20.** N-[3-(3-chloro-5-trifluorométhyl-pyridyl-2-oxy)-4-chlorophényl]-N'-[2,6-difluoro-3-aminobenzoyl]-urée selon la revendication 1.

**21.** N-[3-(3-chloro-5-trifluorométhyl-pyridyl-2-oxy)-4-fluorophényl]-N'-[2,6-difluoro-3-aminobenzoyl]-urée selon la revendication 1.

**22.** N-[3-(3-chloro-5-trifluorométhyl-pyridyl-2-urée selon la revendication 1.

**23.** Composés de formule II

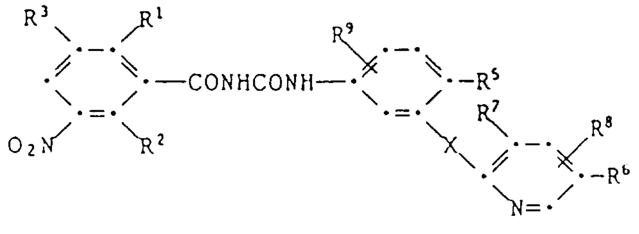

(II)

dans laquelle
$R^1$ , $R^2$ , $R^3$ , $R^7$ $R^8$ et $R^9$ représentent indépendamment l'un de l'autre H ou un halogène, $R^5$ et $R^6$

représentent indépendamment l'un de l'autre H, un halogène, un alkyle en $C_1$ à $C_6$ ou un haloalkyle en $C_1$ à $C_6$ et
X représente O, $S(O)_n$ ou NH, où n vaut 0, 1 ou 2.

**24.** N-[3-(3-chloro-5-trifluorométhyl-pyridyl-2-oxy)-4-chlorophényl]-N'-[2,6-difluoro-3-nitrobenzoyl]-urée, N-[3-(3-chloro-5-trifluorométhyl-pyridyl-2-oxy)-4-fluorophényl]-N'-[2,6-difluoro-3-nitrobenzoyl]-urée et N-[3-(3-chloro-5-trifluorométhyl-pyridyl-é-oxy)-4-chlorophényl]-N'-[2,6-dichloro-3-nitrobenzoyl]-urée selon la revendication 23.

**25.** Composés de formule IV

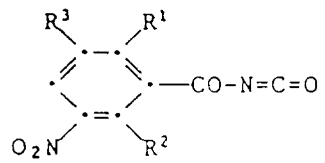

(IV)

dans laquelle
$R^1$ , $R^2$ et $R^3$ représentent indépendamment l'un de l'autre un hydrogène ou un halogène; mais ne représentent pas ensemble un hydrogène.

**26.** Composés de formule VI

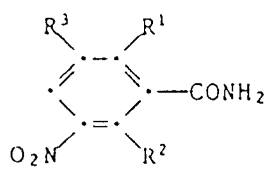

(VI)

dans laquelle
$R^1$ , $R^2$ et $R^3$ représentent indépendamment l'un de l'autre un hydrogène ou un halogène; à l'exception des composés dans lesquels $R^3$ représente un hydrogène et $R^1$ et $R^2$ représentent simultanément un chlore.

**27.** Amide de l'acide 2,6-difluoro-3-nitro-benzoïque selon la revendication 26.

**28.** Procédé de préparation de composés de formule I

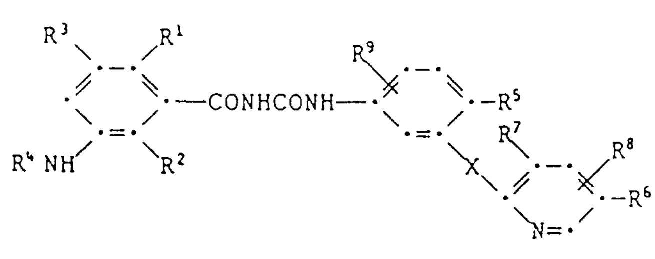

(I)

dans laquelle
$R^1$ , $R^2$ ,$R^3$ , $R^7$ , $R^8$ et $R^9$ représentent indépendamment l'un de l'autre H ou un halogène,
$R^4$ représente H, $R^{10}$ représente CO- ou $R^{11}$ représente NHCO- , où $R^{10}$ représente un alkyle en $C_1$ à $C_4$ , qui est non substitué ou substitué par de un à trois substituants identiques ou différents du groupe constitué par halogène, alcoxy en $C_1$ à $C_4$ , acyloxy en $C_1$ à $C_4$ et -COOG, où G représente H, un cation de métal alcalin ou alcalino-terreux, et $R^{11}$ représente un groupe alkyle en $C_1$ à $C_4$ ou phényle non substitué ou substitué par un halogène,
$R^5$ et $R^6$ représentent indépendamment l'un de l'autre H, un halogène, un alkyle en $C_1$ à $C_6$ ou un

haloalkyle en $C_1$ à $C_6$ , et
X représente O, $S(O)_n$ ou NH, où n vaut 0, 1 ou 2, caractérisé en ce que

a) pour préparer un composé de formule I, dans laquelle $R^4$ représente un hydrogène, i) on hydrogène en présence d'un catalyseur approprié et à pression normale d'hydrogène ou ii) on réduit chimiquement un composé de formule II

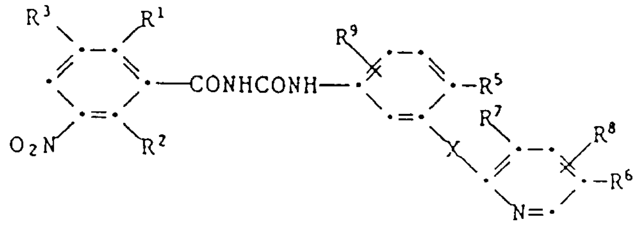

(II)

dans laquelle $R^1$ à $R^9$ et X ont les significations données pour la formule I, ou

b) pour préparer des composés de formule I dans lesquels

$R^4$ représente CO- ou $R^{11}$ représente NHCO- , où $R^{10}$ représente un alkyle en $C_1$ à $C_4$ , qui est non substitué ou substitué par de un à trois substituants identiques ou différents du groupe halogène, alcoxy en $C_1$ à $C_4$ , acyloxy en $C_1$ à $C_4$ et -COOG, où G représente H, un cation de métal alcalin ou alcalino-terreux, et $R^{11}$ représente un groupe alkyle en $C_1$ à $C_4$ ou phényle non substitué ou substitué par un halogène,

on fait réagir un composé de formule I, dans laquelle $R^4$ représente un hydrogène et $R^1$ , $R^2$ , $R^3$ , $R^5$ à $R^9$ et X ont les significations données ci-dessus, avec un acide $R^{10}COOH$, un de ses halogénures d'acide, son anhydride d'acide ou avec un isocyanate $R^{11}NCO$, où $R^{10}$ et $R^{11}$ sont définis comme sous b),

et en ce qu'on peut obtenir les composés de formule II ou bien

(a) en faisant réagir une aniline de formule III

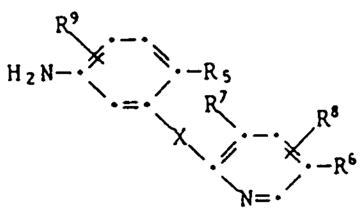

(III)

dans laquelle $R^5$ à $R^9$ et X ont les significations données ci-dessus,
avec un isocyanate de benzoyle de formule IV

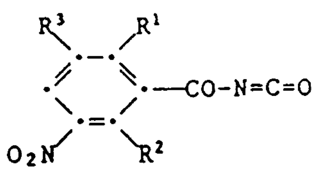

(IV)

dans laquelle $R^1$ , $R^2$ et $R^3$ ont les significations données ci-dessus , ou

(b) en faisant réagir un isocyanate de formule V

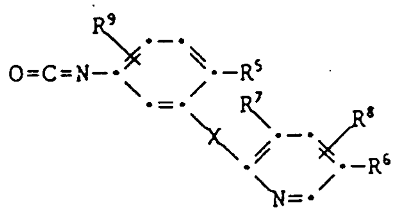

(V)

dans laquelle $R^5$ à $R^9$ et X ont les significations données ci-dessus,
avec un benzamide de formule VI

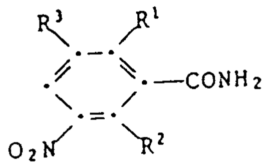

(VI)

dans laquelle $R^1$ , $R^2$ et $R^3$ ont les significations données ci-dessus, ou
(c) en faisant réagir une aniline de formule III avec un uréthane de formule VII

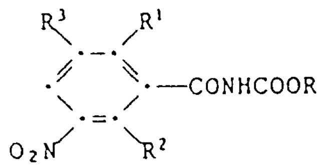

(VII)

dans laquelle $R^1$ , $R^2$ et $R^3$ ont les significations donénes ci-dessus et R représente un alkyle en $C_1$ à $C_5$ ou un phényle non substitué ou substitué par un nitro.

**29.** Agent de lutte contre les ecto- et endoparasites des animaux domestiques et utiles, ou de lutte contre les insectes nuisibles, caractérisé en ce qu'il contient comme matière active, outre les additifs de formulation habituels, un composé de formule I selon la revendication 1.

**30.** Procédé de lutte contre les parasites qui affectent les animaux utiles et les animaux domestiques ou contre les insectes nuisibles, caractérisé en ce qu'on applique une quantité à action parasiticide ou insecticide d'au moins un composé de formule I selon la revendication 1 sur les parasites, les insectes nuisibles ou leur biotope.

**31.** Composés de formule I selon l'une des revendications 1 à 22 pour combattre les parasites chez les animaux utiles ou les animaux domestiques.